# EUROPEAN PATENT APPLICATION

(11) **EP 3 653 216 A1**
(43) Date of publication of application: **20.05.2020**
(21) Application number: 19207672.7
(22) Date of filing: 29.09.2016
(51) Int. Cl.: A61K 31/7125, A61K 31/573, A61K 31/7088, A61P 21/06

(54) **METHODS FOR TREATING MUSCULAR DYSTROPHY**

(30) Priority: 30.09.2015 US 201562235477 P; 14.01.2016 US 201662278866 P; 09.02.2016 US 201662293235 P; 25.02.2016 US 201662299952 P
(62) Divisional of application: 16852633.3
(71) Applicant: Sarepta Therapeutics, Inc., Cambridge, MA 02142 (US)
(72) Inventor: KAYE, Edward, M., Cambridge, MA 02142 (US)
(74) Representative: D Young & Co LLP

(57) **Abstract**

The present disclosure provides, among other things, improved compositions and methods for treating muscular dystrophy. For example, the disclosure provides methods for treating Duchenne muscular dystrophy patients having a mutation in the DMD gene that is amenable to exon 51 skipping by administering an effective amount of eteplirsen.

## Description

### CROSS-REFERENCE TO RELATED APPLICATIONS

This application claims priority to U.S. Provisional Application No. 62/235,477 filed September 30, 2015, U.S. Provisional Application No. 62/278,866 filed January 14, 2016, U.S. Provisional Application No. 62/293,235 filed February 9, 2016, and U.S. Provisional Application No. 62/299,952 filed February 25, 2016, the contents of which are specifically incorporated by reference herein. The contents of any patents, patent applications, and references cited throughout this specification are hereby incorporated by reference in their entireties.

### FIELD OF THE INVENTION

The present invention relates to improved methods for treating muscular dystrophy in a patient with eteplirsen. It also provides compositions suitable for facilitating exon 51 skipping in the human dystrophin gene.

### BACKGROUND OF THE INVENTION

In a variety of genetic diseases, the effects of mutations on the eventual expression of a gene can be modulated through a process of targeted exon skipping during the splicing process. In cases where a normally functional protein is prematurely terminated because of mutations therein, a means for restoring some functional protein production through antisense technology has been shown to be possible through intervention during the splicing processes, and that if exons associated with disease-causing mutations can be specifically deleted from some genes, a shortened protein product can sometimes be produced that has similar biological properties of the native protein or has sufficient biological activity to ameliorate the disease caused by mutations associated with the exon (see *e.g*., Sierakowska, Sambade et al. 1996; Wilton, Lloyd et al. 1999; van Deutekom, Bremmer-Bout et al. 2001; Lu, Mann et al. 2003; Aartsma-Rus, Janson et al. 2004).

Duchenne muscular dystrophy (DMD) is caused by a defect in the expression of the protein dystrophin. The gene encoding the protein contains 79 exons spread out over more than 2 million nucleotides of DNA. Any exonic mutation that changes the reading frame of the exon, or introduces a stop codon, or is characterized by removal of an entire out of frame exon or exons, or duplications of one or more exons, has the potential to disrupt production of functional dystrophin, resulting in DMD.

Disease onset can be documented at birth with elevated creatine kinase levels, and significant motor deficits may be present in the first year of life. By the age of seven or eight, most patients with DMD have an increasingly labored gait and are losing the ability to rise from the floor and climb stairs; by ages 10 to 14, most are wheelchair-dependent. DMD is uniformly fatal; affected individuals typically die of respiratory and/or cardiac failure in their late teens or early 20s. The continuous progression of DMD allows for therapeutic intervention at all stages of the disease; however, treatment is currently limited to glucocorticoids, which are associated with numerous side effects including weight gain, behavioral changes, pubertal changes, osteoporosis, Cushingoid facies, growth inhibition, and cataracts. Consequently, developing better therapies to treat the underlying cause of this disease is imperative.

A less severe form of muscular dystrophy, Becker muscular dystrophy (BMD) has been found to arise where a mutation, typically a deletion of one or more exons, results in a correct reading frame along the entire dystrophin transcript, such that translation of mRNA into protein is not prematurely terminated. If the joining of the upstream and downstream exons in the processing of a mutated dystrophin pre-mRNA maintains the correct reading frame of the gene, the result is an mRNA coding for a protein with a short internal deletion that retains some activity, resulting in a Becker phenotype.

For many years it has been known that deletions of an exon or exons which do not alter the reading frame of a dystrophin protein would give rise to a BMD phenotype, whereas an exon deletion that causes a frame-shift will give rise to DMD (Monaco, Bertelson et al. 1988). In general, dystrophin mutations including point mutations and exon deletions that change the reading frame and thus interrupt proper protein translation result in DMD. It should also be noted that some BMD and DMD patients have exon deletions covering multiple exons.

Recent clinical trials testing the safety and efficacy of splice switching oligonucleotides (SSOs) for the treatment of DMD are based on SSO technology to induce alternative splicing of pre-mRNAs by steric blockade of the spliceosome (Cirak *et al*., 2011; Goemans *et al*., 2011; Kinali *et al*., 2009; van Deutekom *et al*., 2007). However, despite these successes, the pharmacological options available for treating DMD are limited.

Thus, there remains a need for improved compositions and methods for treating muscular dystrophy, such as DMD and BMD in patients.

### SUMMARY OF THE INVENTION

The present disclosure is based, at least in part, on compelling clinical evidence showing that treatment with an exon 51 skipping antisense oligonucleotide, eteplirsen, stabilized walking ability (e.g., stabilization of ambulation), as measured by the 6 Minute Walk Test (6MWT), over a four year period, along with concomitant production of novel dystrophin in muscle of patients with DMD. Specifically, 83% (10/12) of DMD patients chronically treated with eteplirsen at a dose of 30 mg/kg once per week remained ambulant at week 192 with continued stabilization of pulmonary function. In fact, between weeks 168 and 192 of the eteplirsen treatment period, patient ambulation stabilized. This therapeutic efficacy was associated with the observation that all patients had measurable exon 51 skipping at 180 weeks. Furthermore, at 180 weeks, 10 of 11 patients had dystrophin positive fibers as measured by immunohistochemistry and 9 of 11 patients had dystrophin protein expression as measured by western blot analysis. Matched external control patients, that did not receive eteplirsen, had no dystrophin positive fibers as determined by immunohistochemistry and only one of 9 patients had dystrophin protein expression as determined by western blot analysis.

In addition, no drug-related serious adverse events were seen in patients treated with over 2300 doses of eteplirsen over a 192 week treatment period.

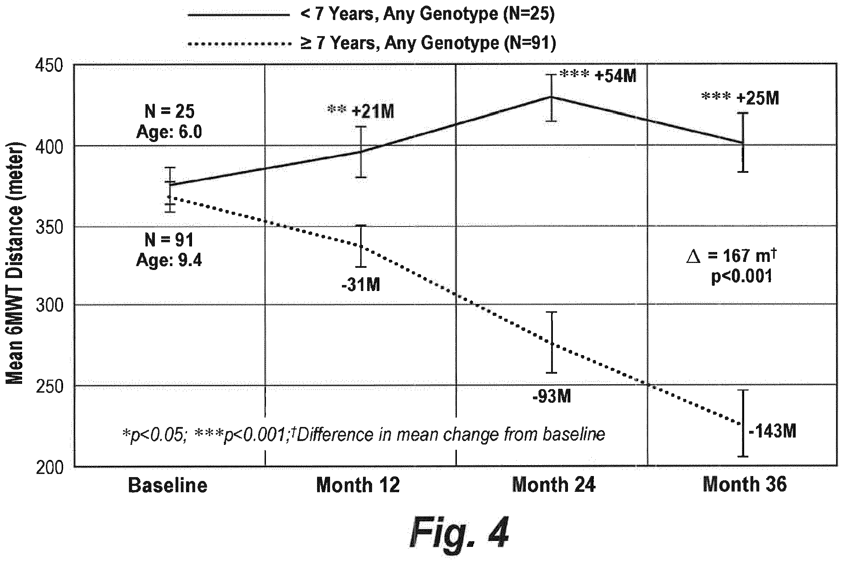

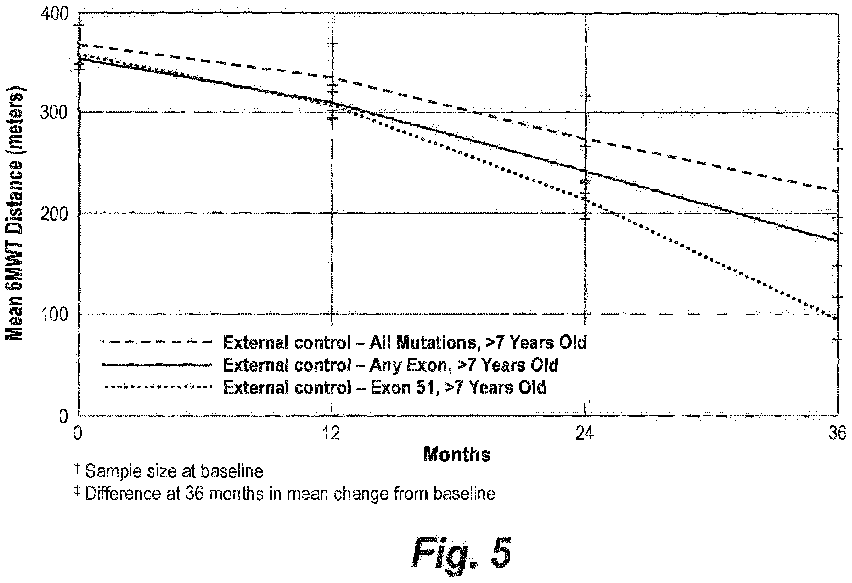

The significance of these unexpected clinical observations with eteplirsen is further magnified by the fact that patients with mutations amenable to exon 51 skipping have a particularly poor prognosis relative to all DMD patients or DMD patients with mutations in any exon of the DMD gene (see Fig. 5). Moreover, eteplirsen was capable of achieving these compelling therapeutic effects in patients seven years of age or older. As shown in Fig. 4, patients in this age group generally suffer a steep decline in ambulation, relative to the rate of decline of ambulation for patients younger than seven. Thus, the present disclosure establishes that eteplirsen treatment can delay disease progression and/or stabilize disease in patient subpopulations having a particularly aggressive form of DMD.

Accordingly, in one aspect, the present invention relates to methods for treating a patient with Duchenne muscular dystrophy (DMD) who has a mutation of the DMD gene that is amenable to exon 51 skipping comprising intravenously administering to the patient eteplirsen at a dose of 30 mg/kg once weekly for more than 168 weeks, thereby treating the patient.

In another aspect, the invention relates to methods for treating a patient with Duchenne muscular dystrophy (DMD) who has a mutation of the DMD gene that is amenable to exon 51 skipping by delaying disease progression, comprising intravenously administering to the patient eteplirsen at a dose of 30 mg/kg once weekly for more than 168 weeks, such that disease progression in the patient is delayed as measured by the 6 Minute Walk Test (6MWT), thereby treating the patient.

In another aspect, the invention relates to methods for maintaining ambulation, or reducing the loss of ambulation, in a patient with Duchenne muscular dystrophy (DMD) who has a mutation of the DMD gene that is amenable to exon 51 skipping, comprising intravenously administering to the patient eteplirsen at a dose of 30 mg/kg once weekly for more than 168 weeks, thereby maintaining ambulation, or reducing the loss of ambulation relative to baseline, in the patient as measured by the 6 Minute Walk Test (6MWT).

In some aspects of the invention, eteplirsen is intravenously administered to the patient once weekly for at least 180 weeks. In other aspects of the invention, eteplirsen is intravenously administered to the patient once weekly for at least 192 weeks.

Other aspects of the invention relate to methods for maintaining ambulation, or reducing the loss of ambulation, in a patient with Duchenne muscular dystrophy (DMD) who has a mutation of the DMD gene that is amenable to exon 51 skipping, comprising intravenously administering to the patient eteplirsen at a dose of 30 mg/kg once weekly for at least 192 weeks, such that the patient maintains ambulation relative to baseline or loses 50 % or less (e.g., 49, 48, 47, 46, 45, 44, or 43 % or less) ambulation, relative to baseline, by 192 weeks as measured by the 6 Minute Walk Test (6MWT).

Other aspects of the invention relate to methods for maintaining ambulation, or reducing the loss of ambulation, in a patient with Duchenne muscular dystrophy (DMD) who has a mutation of the DMD gene that is amenable to exon 51 skipping, comprising intravenously administering to the patient eteplirsen at a dose of 30 mg/kg once weekly for at least 192 weeks, such that the patient maintains ambulation relative to baseline or loses 43 % or less ambulation, relative to baseline, by 192 weeks as measured by the 6 Minute Walk Test (6MWT).

In another aspect, the invention relates to methods for maintaining pulmonary function or reducing loss of pulmonary function in a patient with Duchenne muscular dystrophy (DMD) who has a mutation of the DMD gene that is amenable to exon 51 skipping, comprising intravenously administering to the patient eteplirsen at a dose of 30 mg/kg once weekly for more than 168 weeks, thereby maintaining pulmonary function, or reducing the loss of pulmonary function, in the patient relative to baseline.

In these and other aspects of the invention, pulmonary function is measured as Maximum Expiratory Pressure (MEP). In these and other aspects of the invention, pulmonary function is measured as Maximum Inspiratory Pressure (MIP). In these and other aspects of the invention, pulmonary function is measured as Forced Vital Capacity (FVC).

Other aspects of the invention relate to methods for restoring an mRNA reading frame to induce dystrophin protein production in a patient with Duchenne muscular dystrophy (DMD) who has a mutation of the DMD gene that is amenable to exon 51 skipping, comprising intravenously administering to the patient eteplirsen at a dose of 30 mg/kg once weekly for more than 168 weeks, thereby restoring the mRNA reading frame to induce dystrophin protein production in the patient.

In these and other aspects of the invention dystrophin protein production can be measured by, e.g., reverse transcription polymerase chain reaction (RT-PCR), western blot analysis, or immunohistochemistry (IHC).

Another aspect of the invention relates to methods for treating a patient with Duchenne muscular dystrophy (DMD) who has a mutation of the DMD gene that is amenable to exon 51 skipping by delaying disease progression, comprising intravenously administering to the patient eteplirsen at a dose of 30 mg/kg once weekly for more than 168 weeks, such that disease progression in the patient is delayed, thereby treating the patient.

Other aspects of the invention relate to methods for maintaining ambulation, or reducing the loss of ambulation, in a patient with Duchenne muscular dystrophy (DMD) who has a mutation of the DMD gene that is amenable to exon 51 skipping, comprising intravenously administering to the patient eteplirsen at a dose of 30 mg/kg once weekly for more than 168 weeks, thereby maintaining ambulation, or reducing the loss of ambulation relative to baseline, in the patient.

In these and other aspects of the invention, eteplirsen is intravenously administered to the patient once weekly for at least 180 weeks or at least 192 weeks.

Another aspect of the invention relates to methods for maintaining ambulation, or reducing the loss of ambulation, in a patient with Duchenne muscular dystrophy (DMD) who has a mutation of the DMD gene that is amenable to exon 51 skipping, comprising intravenously administering to the patient eteplirsen at a dose of 30 mg/kg once weekly for at least 192 weeks, such that the patient maintains ambulation relative to baseline or loses 43% or less ambulation, relative to baseline, by 192 weeks.

In these and other aspects of the invention ambulation is measured using the North Star Ambulatory Assessment (NSAA).

Other aspects of the invention relate to eteplirsen for use in treating a patient with Duchenne muscular dystrophy (DMD) who has a mutation of the DMD gene that is amenable to exon 51 skipping, wherein eteplirsen is to be intravenously administered to the patient at a dose of 30 mg/kg once weekly for more than 168 weeks.

In another aspect, the invention relates to eteplirsen for use in treating a patient with Duchenne muscular dystrophy (DMD) who has a mutation of the DMD gene that is amenable to exon 51 skipping by delaying disease progression, wherein eteplirsen is to be intravenously administered to the patient at a dose of 30 mg/kg once weekly for more than 168 weeks, such that disease progression in the patient is delayed as measured by the 6 Minute Walk Test (6MWT).

In another aspect, the invention relates to eteplirsen for use in maintaining ambulation, or reducing the loss of ambulation, in a patient with Duchenne muscular dystrophy (DMD) who has a mutation of the DMD gene that is amenable to exon 51 skipping, wherein eteplirsen is to be intravenously administered to the patient at a dose of 30 mg/kg once weekly for more than 168 weeks, thereby maintaining ambulation, or reducing the loss of ambulation relative to baseline, in the patient as measured by the 6 Minute Walk Test (6MWT).

Another aspect of the invention relates to eteplirsen for use in maintaining ambulation, or reducing the loss of ambulation, in a patient with Duchenne muscular dystrophy (DMD) who has a mutation of the DMD gene that is amenable to exon 51 skipping, wherein eteplirsen is to be intravenously administered to the patient at a dose of 30 mg/kg once weekly for at least 192 weeks, such that the patient maintains ambulation relative to baseline or loses 43% or less ambulation, relative to baseline, by 192 weeks.

In another aspect, the invention relates to eteplirsen for use in maintaining pulmonary function or reducing loss of pulmonary function in a patient with Duchenne muscular dystrophy (DMD) who has a mutation of the DMD gene that is amenable to exon 51 skipping, wherein eteplirsen is to be intravenously administered to the patient at a dose of 30 mg/kg once weekly for more than 168 weeks, thereby maintaining pulmonary function, or reducing the loss of pulmonary function, in the patient relative to baseline.

Other aspects of the invention relate to eteplirsen for use in restoring an mRNA reading frame to induce dystrophin protein production in a patient with Duchenne muscular dystrophy (DMD) who has a mutation of the DMD gene that is amenable to exon 51 skipping, wherein eteplirsen is to be intravenously administered to the patient at a dose of 30 mg/kg once weekly for more than 168 weeks, thereby restoring the mRNA reading frame to induce dystrophin protein production in the patient.

Another aspect of the invention relates to eteplirsen for use in treating a patient with Duchenne muscular dystrophy (DMD) who has a mutation of the DMD gene that is amenable to exon 51 skipping by delaying disease progression, wherein eteplirsen is to be intravenously administered to the patient at a dose of 30 mg/kg once weekly for more than 168 weeks, such that disease progression in the patient is delayed, thereby treating the patient.

Other aspects of the invention relate to eteplirsen for use in maintaining ambulation, or reducing the loss of ambulation, in a patient with Duchenne muscular dystrophy (DMD) who has a mutation of the DMD gene that is amenable to exon 51 skipping, wherein eteplirsen is to be intravenously administered to the patient at a dose of 30 mg/kg once weekly for more than 168 weeks, thereby maintaining ambulation, or reducing the loss of ambulation relative to baseline, in the patient.

Another aspect of the invention relates to eteplirsen for use in maintaining ambulation, or reducing the loss of ambulation, in a patient with Duchenne muscular dystrophy (DMD) who has a mutation of the DMD gene that is amenable to exon 51 skipping, wherein eteplirsen is to be intravenously administered to the patient at a dose of 30 mg/kg once weekly for at least 192 weeks, such that the patient maintains ambulation relative to baseline or loses 43% or less ambulation, relative to baseline, by 192 weeks.

In any of the foregoing and other aspects of the invention, the patient with DMD is seven years of age or older.

In any of the foregoing and other aspects of the invention, eteplirsen is administered as an intravenous infusion over 35 to 60 minutes.

In any of the foregoing and other aspects of the invention, the patient is administered a corticosteroid (e.g., prednisone) in addition to administration of eteplirsen. A corticosteroid can be administered prior to treatment with eteplirsen or in conjunction with eteplirsen treatment or subsequent to treatment with eteplirsen.

In any of the foregoing and other aspects of the invention, the methods of the invention further comprise confirming that the patient has a mutation in the DMD gene that is amenable to exon 51 skipping.

The present disclosure also provides compelling clinical evidence showing that 10 out of 12 (83.3%) patients treated with eteplirsen remained ambulant at 216 weeks (over 4 years). By contrast, 89.7% of patients in a matched, external control cohort lost ambulation, as measured by Kaplan-Meier analysis (see the working examples). In fact, the Kaplan-Meier estimate for loss of ambulation rate at 4 years of treatment is 16.7% for eteplirsen-treated patients, and 89.7% for the external control (p=0.0038, log-rank test).

Moreover, as shown in Tables 2 and 3 below, not only did 10 out of 12 patients remain ambulant over the course of 216 weeks of treatment, at ages in which loss of ambulation is expected, but, in some cases, patients a retained a considerable percentage of their walking ability, as measured by the 6 Minute Walk Test (6MWT). For example, subject number 15 lost one meter in the 6MWT at 216 weeks, relative to baseline (Table 2). In another example, several eteplirsen-treated subjects, including subject number 4, retained greater than 50% of their walking ability, relative to baseline. These data show that eteplirsen treatment is highly effective at treating patients with Duchenne muscular dystrophy, including, among other uses, delaying disease progression, reducing the severity of disease progression, reducing the rate of loss of ambulation, maintaining ambulation, and reducing loss of ambulation in such patients.

Therefore, in another aspect, the disclosure provides methods for treating a patient with Duchenne muscular dystrophy (DMD) in need thereof who has a mutation of the DMD gene that is amenable to exon 51 skipping by delaying disease progression. The method comprises intravenously administering to the patient eteplirsen at a dose of 30 mg/kg weekly for more than 192 weeks (e.g., 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 230, 235, 240, 245, 250, 255, or 260 or more), such that disease progression in the patient is delayed as measured by the 6 Minute Walk Test (6MWT), thereby treating the patient.

Other aspects of the invention relate to methods for treating a patient with Duchenne muscular dystrophy (DMD) in need thereof who has a mutation of the DMD gene that is amenable to exon 51 skipping by reducing the severity of disease progression, comprising intravenously administering to the patient eteplirsen at a dose of 30 mg/kg weekly for more than 192 weeks, such that the severity of disease progression in the patient is reduced as measured by the 6 Minute Walk Test (6MWT), thereby treating the patient.

In another aspect, the invention relates to methods for treating a patient with Duchenne muscular dystrophy (DMD) in need thereof who has a mutation of the DMD gene that is amenable to exon 51 skipping by reducing the rate of loss of ambulation in the patient, comprising intravenously administering to the patient eteplirsen at a dose of 30 mg/kg weekly for more than 192 weeks, such that the rate of loss of ambulation in the patient is no greater than 20% at 216 weeks, as measured by the 6 Minute Walk Test (6MWT), thereby treating the patient.

Another aspect of the invention relates to methods for maintaining ambulation, or reducing the loss of ambulation, in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly for more than 192 weeks, thereby maintaining ambulation, or reducing the loss of ambulation relative to baseline, in the patient as measured by the 6 Minute Walk Test (6MWT).

In these and other aspects of the invention, ambulation is maintained for at least 216 weeks of treatment.

In these and other aspects of the invention, the patient's loss of ambulation between about three years of treatment and about four years of treatment is no greater than 20%. In other aspects of the invention, the patient's loss of ambulation between about three years of treatment and about four years of treatment is no greater than 30%. In yet other aspects of the invention, the patient's loss of ambulation between about three years of treatment and about four years of treatment is no greater than 40%. In other aspects of the invention, the patient's loss of ambulation between about three years of treatment and about four years of treatment is no greater than 50%.

In these and other aspects of the invention, the patient's loss of ambulation is no greater than about 20% relative to base line. In other aspects of the invention, the patient's loss of ambulation is no greater than about 30% relative to base line. In yet others aspects of the invention, the patient's loss of ambulation is no greater than about 40% relative to base line. In other aspects of the invention, the patient's loss of ambulation is no greater than about 50% relative to base line. In other aspects of the invention, the patient's loss of ambulation is no greater than about 60% relative to base line.

Another aspect of the invention relates to methods for maintaining ambulation in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly for more than 192 weeks, such that the patient remains ambulant at 216 weeks, as measured by the 6 Minute Walk Test (6MWT).

In these and other aspects of the invention, eteplirsen is intravenously administered to the patient weekly for at least 216 weeks.

In these and other aspects of the invention, the patient is seven years of age or older (e.g., at the start of eteplirsen treatment). In another aspect of the invention, the patient is 11 years of age or older at 216 weeks of treatment with eteplirsen. In yet other aspects of the invention, the patient is 14 years of age or older at 216 weeks of treatment with eteplirsen.

In these and other aspects of the invention, eteplirsen is administered as an intravenous infusion over 35 to 60 minutes.

In these and other aspects of the invention, the patient maintains a 6 Minute Walk Distance (6MWD) of at least 55 meters at 216 weeks of treatment. In other aspects of the invention, the patient maintains a 6 Minute Walk Distance (6MWD) of at least 100 meters at 216 weeks of treatment. In yet other aspects of the invention, the patient maintains a 6 Minute Walk Distance (6MWD) of at least 200 meters at 216 weeks of treatment. In other aspects of the invention, the patient maintains a 6 Minute Walk Distance (6MWD) of at least 300 meters at 216 weeks of treatment. In another aspects of the invention, the patient maintains a 6 Minute Walk Distance (6MWD) of at least 400 meters at 216 weeks of treatment.

In these and other aspects of the invention, the patient is administered a steroid in addition to administration of eteplirsen. In other aspects of the invention, the steroid is administered to the patient prior to, in conjunction with, or subsequent to administration of eteplirsen.

In these and other aspects of the invention, the patient is on background steroid therapy. In other aspects of the invention, the steroid is a corticosteroid. In yet other aspects of the invention, the corticosteroid is betamethasone, budesonide, cortisone, dexamethasone, hydrocortisone, methylprednisolone, prednisone, prednisolone, or deflazacort.

In these and other aspects of the invention, the methods of the invention further comprise confirming that the patient has a mutation in the DMD gene that is amenable to exon 51 skipping.

Another aspect of the invention relates to methods for restoring an mRNA reading frame to induce dystrophin protein production in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly for at least 216 weeks, thereby restoring the mRNA reading frame to induce dystrophin protein production in the patient.

In these and other aspects of the invention, the dystrophin protein production is measured by reverse transcription polymerase chain reaction (RT-PCR), western blot analysis, or immunohistochemistry (IHC).

Other aspects of the invention relates to methods for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly for at least 216 weeks, such that disease progression in the patient is delayed, thereby treating the patient.

Another aspect of the invention relates to methods for maintaining ambulation, or reducing the loss of ambulation, in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly for more than 192 weeks, thereby maintaining ambulation, or reducing the loss of ambulation relative to baseline, for at least 216 weeks in the patient.

In another aspect, the invention relates to methods for maintaining pulmonary function or reducing loss of pulmonary function in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly for more than 192 weeks, thereby maintaining pulmonary function, or reducing the loss of pulmonary function, in the patient relative to baseline for at least 216 weeks.

In these and other aspects of the invention, pulmonary function is measured as Maximum Expiratory Pressure (MEP). In other aspects of the invention, pulmonary function is measured as Maximum Inspiratory Pressure (MIP). In yet other aspects of the invention, pulmonary function is measured as Forced Vital Capacity (FVC).

In some embodiments of any of the methods described herein, the patient is one on background steroid therapy.

Some literature reports suggest an association in DMD patients between loss of ability to rise from the floor independently and a subsequent loss of ambulation within the following year (Bushby and Connor (2011) Clin Investig (Lond.) 1(9):1217-1235 and Henricson et al. (2013) Muscle Nerve 48(1):55-67). As described in the working examples below, four eteplirsen-treated patients lost the ability to rise without external support from years 1 to 3. Yet, despite the loss of ability to rise from supine, these eteplirsen patients remained ambulant at year 4 of treatment. Thus, these data indicate that eteplirsen-treatment preserves ambulatory ability even in patients who have lost the ability to rise independently from supine.

Accordingly, in yet another aspect, any of the methods described herein may involve treating a patient who has lost the ability to rise independently from supine. In some embodiments of any of the methods described herein, the patient loses the ability to rise independently from supine at least one year prior to treatment with eteplirsen. In some embodiments of any of the methods described herein, the patient loses the ability to rise independently from supine within one year of beginning treatment with eteplirsen. In some embodiments of any of the methods described herein, the patient loses the ability to rise independently from supine within two years of beginning treatment.

In some embodiments, any of the methods described herein comprise continuing treatment with eteplirsen even if a patient loses the ability to rise from a supine position during treatment with eteplirsen.

In some embodiments of any of the methods described herein, the patient has a rise time of greater than 10 seconds. In some embodiments of any of the methods described herein, the patient has a rise time of greater than 15 seconds. In some embodiments of any of the methods described herein, the patient has a rise time of greater than 20 seconds.

In another aspect, the invention provides a method for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly, such that disease progression in the patient is delayed as measured by the 6 Minute Walk Test (6MWT), thereby treating the patient, wherein the patient has lost the ability to rise independently from supine prior to treatment with eteplirsen.

In another aspect, the invention features a method for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly, such that disease progression in the patient is delayed as measured by the 6 Minute Walk Test (6MWT), thereby treating the patient, wherein the patient loses the ability to rise independently from supine during treatment with eteplirsen (e.g., at about 6 months, a year, two years, or even three years after beginning treatment with eteplirsen).

In yet another aspect, the invention features a method for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly, such that disease progression in the patient is delayed as measured by the 6 Minute Walk Test (6MWT), thereby treating the patient, wherein the patient has lost the ability to rise independently from supine prior to treatment with eteplirsen.

In another aspect, the invention features a method for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly, such that disease progression in the patient is delayed as measured by the 6 Minute Walk Test (6MWT), thereby treating the patient, wherein the patient loses the ability to rise independently from supine during treatment with eteplirsen (e.g., at about 6 months, a year, two years, or even three years after beginning treatment with eteplirsen).

In another aspect, the invention features a method for maintaining ambulation, or reducing the loss of ambulation, in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby maintaining ambulation, or reducing the loss of ambulation relative to baseline, in the patient as measured by the 6 Minute Walk Test (6MWT), wherein the patient has lost the ability to rise independently from supine prior to treatment with eteplirsen.

In another aspect, the invention features a method for maintaining ambulation, or reducing the loss of ambulation, in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby maintaining ambulation, or reducing the loss of ambulation relative to baseline, in the patient as measured by the 6 Minute Walk Test (6MWT), wherein the patient loses the ability to rise independently from supine during treatment with eteplirsen (e.g., at about 6 months, a year, two years, or even three years after beginning treatment with eteplirsen).

In another aspect, the invention features a method for maintaining pulmonary function or reducing loss of pulmonary function in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby maintaining pulmonary function, or reducing the loss of pulmonary function, in the patient relative to baseline, wherein the patient has lost the ability to rise independently from supine prior to treatment with eteplirsen.

In another aspect, the invention features a method for maintaining pulmonary function or reducing loss of pulmonary function in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby maintaining pulmonary function, or reducing the loss of pulmonary function, in the patient relative to baseline, wherein the patient loses the ability to rise independently from supine during treatment with eteplirsen (e.g., at about 6 months, a year, two years, or even three years after beginning treatment with eteplirsen).

In some embodiments of any of the methods described herein, pulmonary function is measured as Maximum Expiratory Pressure (MEP). In some embodiments, pulmonary function is measured as Maximum Inspiratory Pressure (MIP). In some embodiments, pulmonary function is measured as Forced Vital Capacity (FVC).

In another aspect, the invention features a method for restoring an mRNA reading frame to induce dystrophin protein production in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby restoring the mRNA reading frame to induce dystrophin protein production in the patient, wherein the patient has lost the ability to rise independently from supine prior to treatment with eteplirsen.

In another aspect, the invention features a method for restoring an mRNA reading frame to induce dystrophin protein production in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby restoring the mRNA reading frame to induce dystrophin protein production in the patient, wherein the patient loses the ability to rise independently from supine during treatment with eteplirsen (e.g., at about 6 months, a year, two years, or even three years after beginning treatment with eteplirsen).

In some embodiments, the dystrophin protein production is measured by reverse transcription polymerase chain reaction (RT-PCR), western blot analysis, or immunohistochemistry (IHC).

In some embodiments of any of the methods described herein, the patient is seven years of age or older. In some embodiments of any of the methods described herein, eteplirsen is administered as an intravenous infusion over 35 to 60 minutes.

In some embodiments, any of the methods described herein comprise administering to the patient a steroid, e.g., a corticosteroid. In some embodiments, the corticosteroid is Betamethasone, Budesonide, Cortisone, Dexamethasone, Hydrocortisone, Methylprednisolone, Prednisolone, Prednisone, or deflazacort.

In some embodiments of any of the methods described herein, the corticosteroid is administered prior to, in conjunction with, or subsequent to administration of eteplirsen.

In some embodiments of any of the methods described herein, the patient is on background steroids (e.g., intermittent or continuous/chronic background steroid therapy).

In some embodiments, any of the methods described herein further comprise confirming that the patient has a mutation in the DMD gene that is amenable to exon 51 skipping.

In some embodiments, any of the methods described herein further comprise confirming that the patient has a mutation in the DMD gene that is amenable to exon 51 skipping prior to administering eteplirsen.

In some embodiments of any of the methods described herein, the patient loses the ability to rise independently from supine within one year after beginning treatment with eteplirsen. In some embodiments of any of the methods described herein, the patient loses the ability to rise independently from supine within two years after beginning treatment. In some embodiments of any of the methods described herein, the patient has a rise time of greater than 10 seconds at the beginning of treatment. In some embodiments of any of the methods described herein, the patient has a rise time of greater than 15 seconds at the beginning of treatment. In some embodiments of any of the methods described herein, the patient has a rise time of greater than 20 seconds.

In some embodiments of any of the methods described herein, the patient is on intermittent background steroid therapy. In some embodiments of any of the methods described herein, the patient is on continuous background steroid therapy.

In some embodiments of any of the methods or compositions described herein, the patient is between about 6 months and about 4 years of age, inclusive. In some embodiments, the patient is at least 6 (e.g., at least 7, 8, 9, 10, 11, 12, 13, 14, 15, 16, 17, 18, 19, 20, 24, 30, or 36) months of age. In some embodiments, the patient is no greater than 4 years of age. In some embodiments, eteplirsen may be administered to such patients parenterally, e.g., subcutaneously.

In yet another aspect, the disclosure features a method for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly, in conjunction with a steroid, such that disease progression in the patient is delayed as measured by the 6 Minute Walk Test (6MWT), thereby treating the patient, wherein administration of eteplirsen allows for a lower dose of the steroid to be administered to the patient, relative to the CDC/TREAT-NMD guideline recommended dose of the steroid (Bushby K, Lynn S, Straub V. Collaborating to bring new therapies to the patient: the TREAT-NMD model. Acta Myo 2009;28:12-15), and achieve a comparable, the same, or an increased level of efficacy. For example, the dose of steroid administered to the patient can be between about 75% and about 80% of the recommended dosage of the steroid.

In another aspect, the invention relates to Eteplirsen for use in treating Duchenne muscular dystrophy (DMD) in a patient in need thereof, the patient having a mutation of the DMD gene that may be corrected by skipping exon 51, wherein the treatment comprises administering to the patient a single intravenous dose of eteplirsen of 30 mg/kg once a week, thereby delaying the progression of the disease over 168 weeks as determined by the 6 Minute Walk Test (6MWT)

In another aspect, the invention relates to Eteplirsen for use in maintaining ambulation, or reducing loss of ambulation in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby maintaining ambulation or reducing loss of ambulation relative to baseline over 168 weeks as determined by the 6 Minute Walk Test (6MWT)

Other aspects of the inventions relate to Eteplirsen for use in maintaining pulmonary function or reducing loss of pulmonary function in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby maintaining pulmonary function or reducing the loss of pulmonary function relative to baseline over 168 weeks.

In another aspect, the invention relates to Eteplirsen for use in restoring an mRNA reading frame to induce dystrophin protein production in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby restoring the mRNA reading frame to induce dystrophin protein production over 168 weeks.

Another aspect of the invention relates to Eteplirsen for use in restoring an mRNA reading frame to induce dystrophin protein production in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby restoring the mRNA reading frame to induce dystrophin protein production for at least 216 weeks.

### BRIEF DESCRIPTION OF THE FIGURES

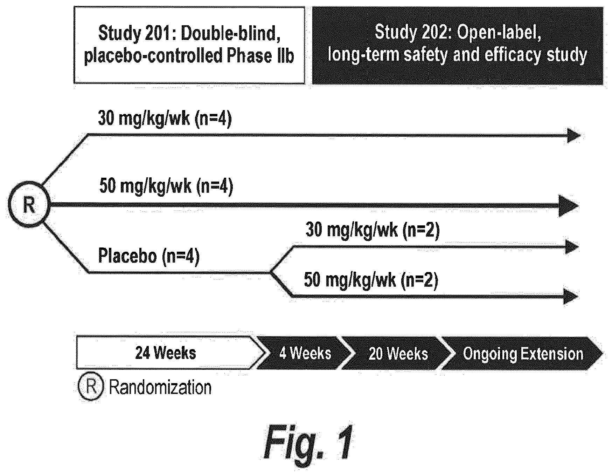

**FIG.1** is a schematic representation of the study design for treating DMD patients. Twelve DMD patients were randomized to one of three cohorts in the double-blind, placebo-controlled study, 201: Cohort 1, eteplirsen 30 mg/kg/wk; Cohort 2, eteplirsen 50 mg/kg/wk; and Cohort 3, placebo/delayed eteplirsen. At week 25, placebo-treated patients in Cohort 3 switched to open-label treatment with 30 or 50 mg/kg/week eteplirsen. Patients were maintained on their same dose of eteplirsen under the open-label extension study, 202. **Efficacy Evaluations.** The 6MWT was used as a functional outcome measure.

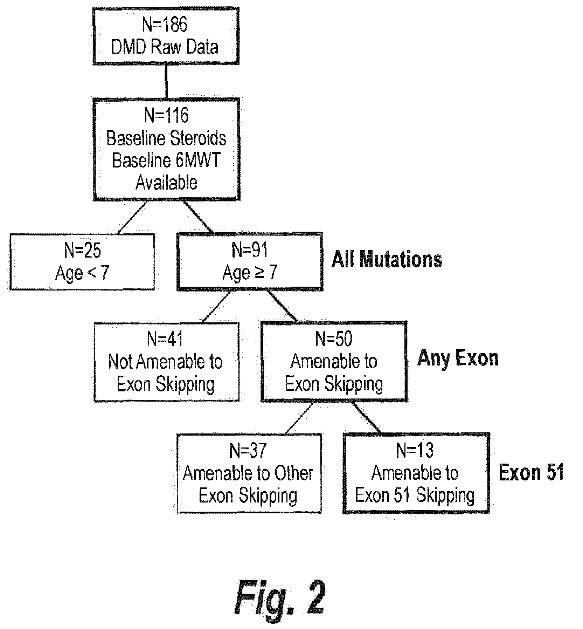

**FIG. 2** is a schematic representation of the selection of a matched external control cohort. The Italian Telethon (n=97) and Leuven NMRC (n=89) registries were selected for having 6MWT data in exon 51 amenable patients, longitudinal 6MWT data up to 36 months, genetic mutation data, and equivalent care standards. The factors for selecting patients were: steroid treated, >7 years old, exon 51 skipping amenable, and a baseline 6MWT of 180-400m. This resulted in 13 patients being selected as matched controls.

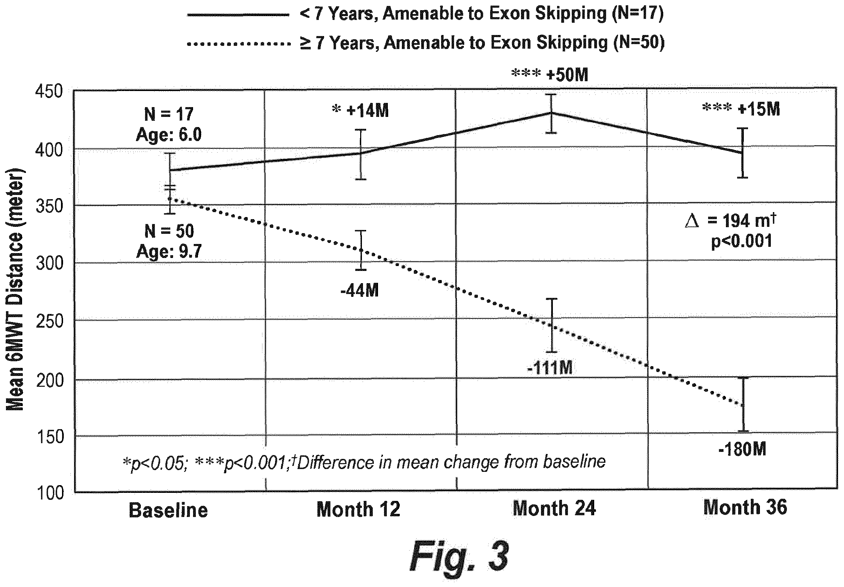

**FIG. 3** graphically depicts the results of the 6MWT in distance over time during the natural history of disease progression in DMD patients amenable to exon skipping based on age groups (i.e., younger than 7 year of age or 7 years of age and older). The dark line shows the change in distance in patients younger than 7 years of age. The light line shows the change in distance in patients 7 years of age or older. A difference of 194 m is noted at the end of 36 months.
**FIG. 4** graphically depicts the results of the 6MWT in distance over time during the natural history of disease progression in DMD patients having any genotype based on age groups (i.e., younger than 7 year of age or 7 years of age and older). The dark line shows the change in distance in patients younger than 7 years of age. The light line shows the change in distance in patients 7 years of age or older. A difference of 167 m is noted at the end of 36 months.
**FIG. 5** graphically depicts the results of the 6MWT in distance over time during the natural history of disease progression in DMD patients based on genotype. The upper line shows the change in distance in patients having all mutations. The middle line shows the change in distance in patients having mutations in any exon. The lower line shows the change in distance in patients having exon 51 mutations.

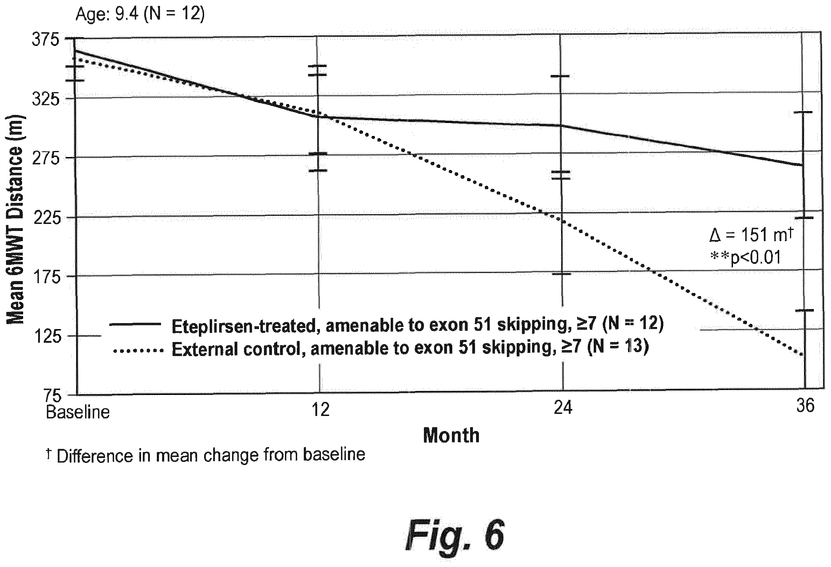

**FIG. 6** graphically depicts the functional efficacy of eteplirsen. The dark line shows the change in distance (m) from baseline in distance walked on the 6MWT over time for the 12 patients who received eteplirsen, either from the start of the study or those who received placebo first. The lighter line shows the change from baseline in distance walked on the 6MWT over time for the 13 matched control patients. A difference of 151 m is noted at the end of 36 months.

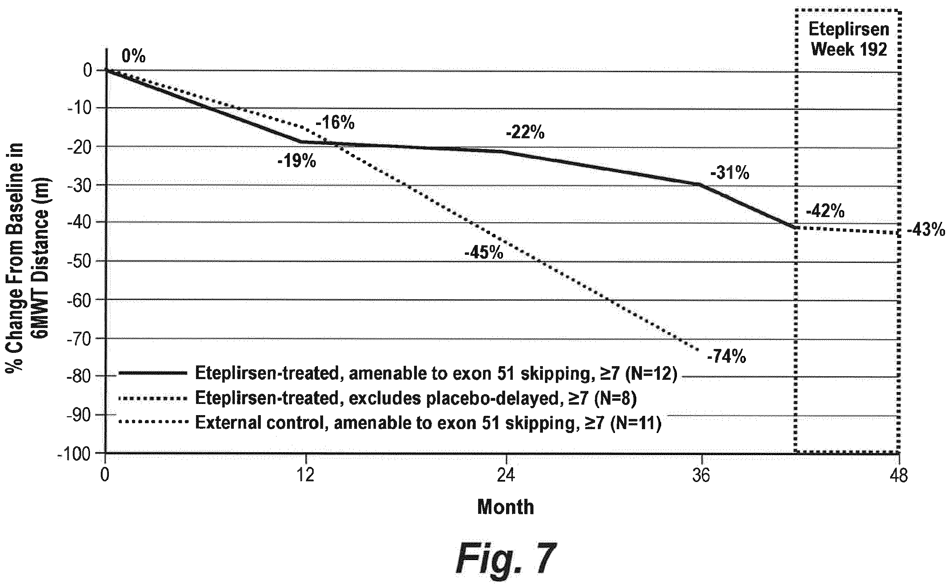

**FIG. 7** graphically depicts the results of the 6MWT as percent change from baseline over time. The dark line represents the 12 patients who received eteplirsen, either from the start of the study or those who received placebo first. The dashed line shows the change in the 8 patients who were not placebo-delayed. The lighter line represents the 11 matched control patients.

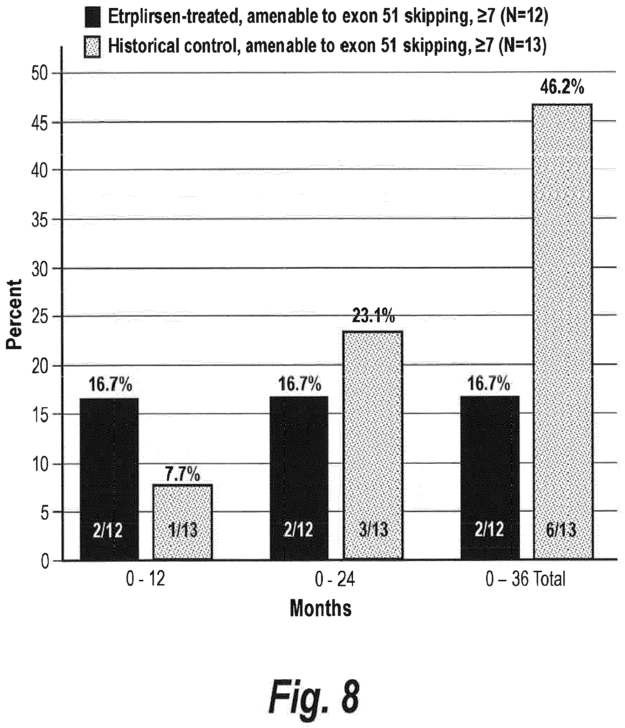

**FIG. 8** graphically depicts the percent of patients that lost ambulation over time. The dark bar represents the 12 patients who received eteplirsen and the lighter bar represents the 13 matched control patients.

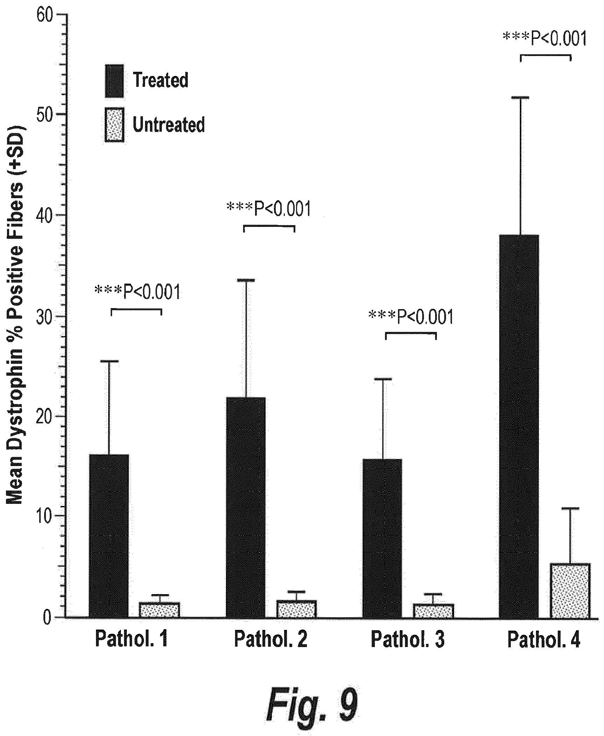

**FIG. 9** graphically depicts the percent of dystrophin positive fibers in both the eteplirsen-treated cohort ("treated") and the matched external control cohort ("untreated") at 180 weeks from baseline. Four independent pathologists looked at tissue sections and determined the percentage of dystrophin positive fibers.

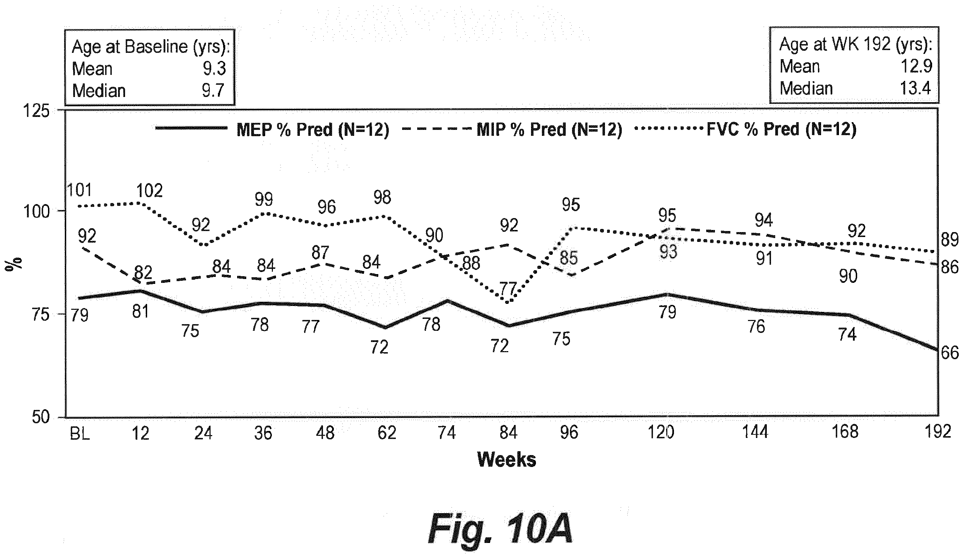

**FIG. 10A** shows the progression of pulmonary function in the eteplirsen-treated cohort. The uppermost line represents force vital capacity (FVC) as a percentage. The middle line shows the maximum inspiratory pressure (MIP) as a percentage and the lower line shows the maximum expiratory pressure (MEP) as a percentage.

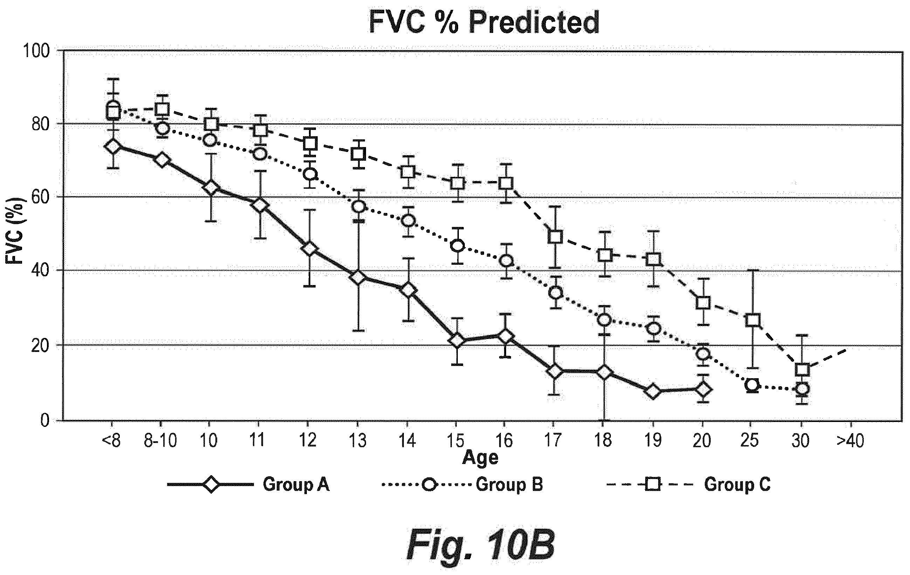

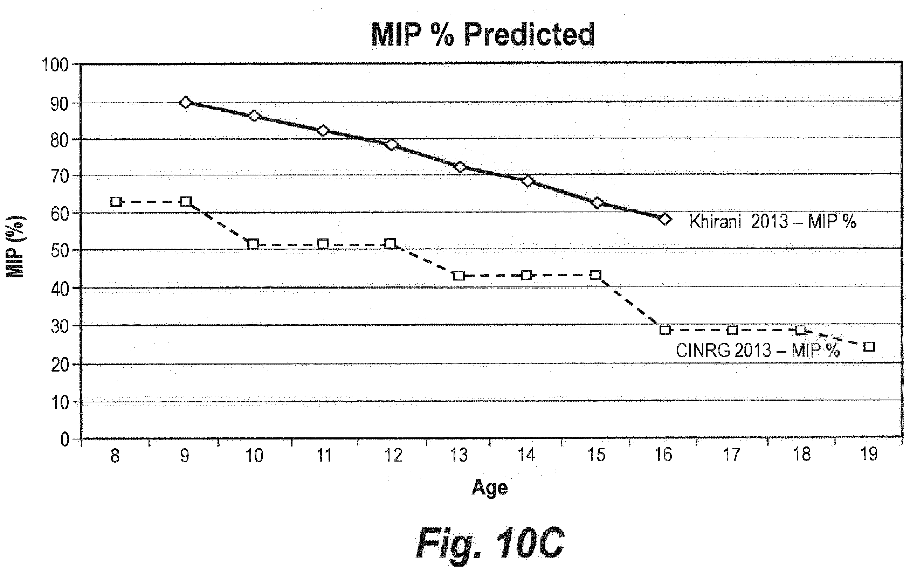

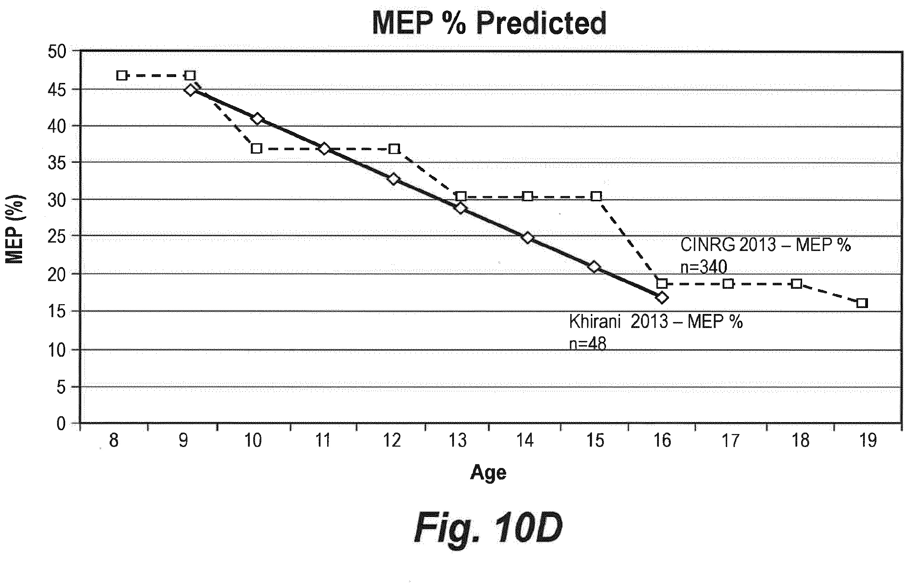

**FIGs. 10B-10D** show the progression of pulmonary function in the matched external control cohort. **FIG. 10B** shows force vital capacity (FVC) as a percentage. **FIG. 10C** shows maximum inspiratory pressure (MIP) as a percentage. **FIG. 10D** shows maximum expiratory pressure (MEP) as a percentage.

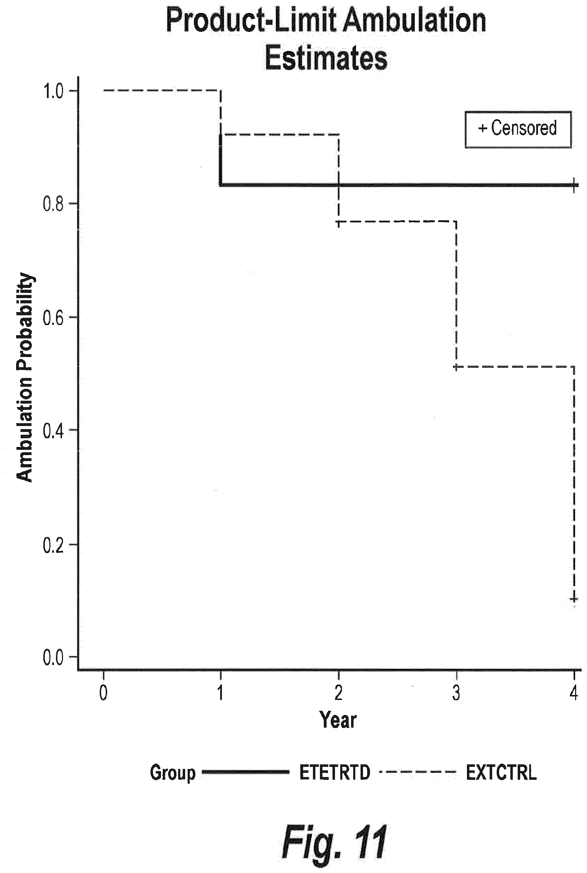

**FIG.11** graphically depicts the probability of ambulation in both the eteplirsen-treated cohort ("ETETRTD") and the matched external control cohort ("EXTCTRL") as determined by a Kaplan-Meier curve, over 4 years. The estimated loss of ambulation is 16.7% for the ETETRTD cohort and 89.7% for the EXTCTRL cohort. P=0.0038 based on log-rank test.

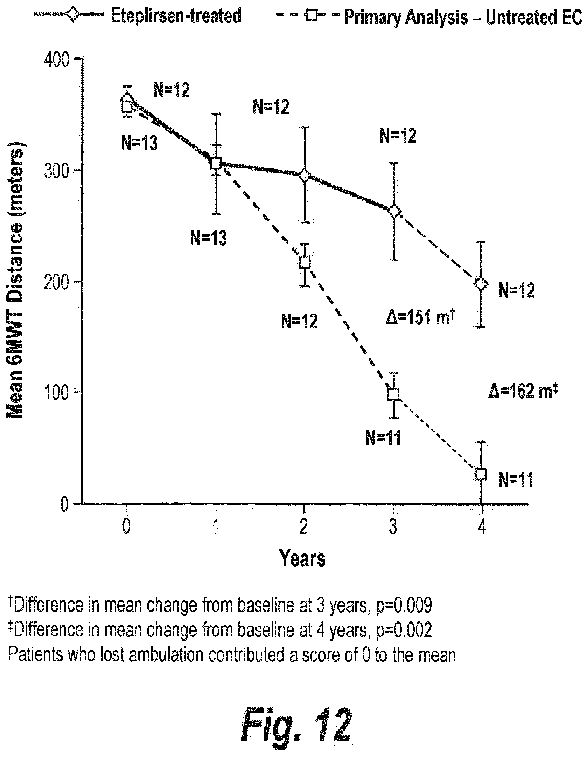

**FIG. 12** graphically depicts the functional efficacy of eteplirsen. The upper line (diamonds) shows the mean change in distance (m) from baseline in distance walked on the 6MWT over time for the 12 patients who received eteplirsen, either from the start of the study or those who received placebo first. The lower line (squares) shows the mean change from baseline in distance walked on the 6MWT over time for the 13 matched control patients. A difference of 162 m is noted at the end of 4 years.

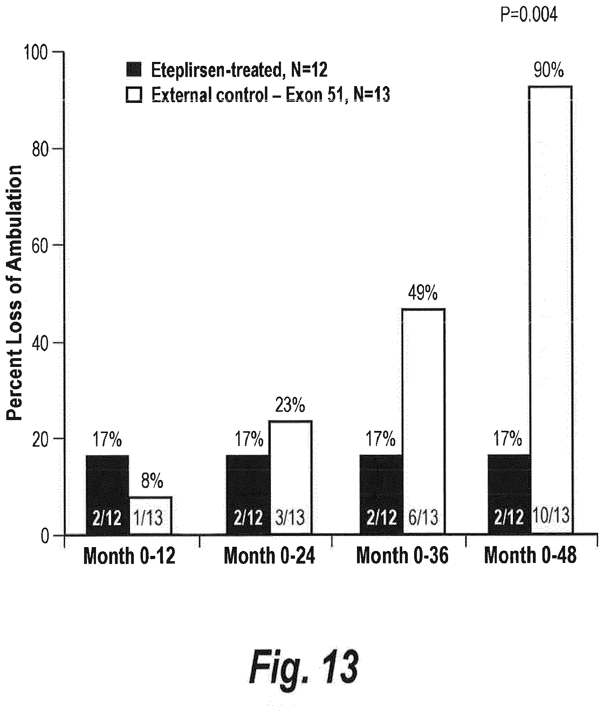

**FIG.13** is a bar graph depicting the percentage of patients in each cohort (eteplirsen-treated and the matched external control cohorts) who have lost ambulation by time.

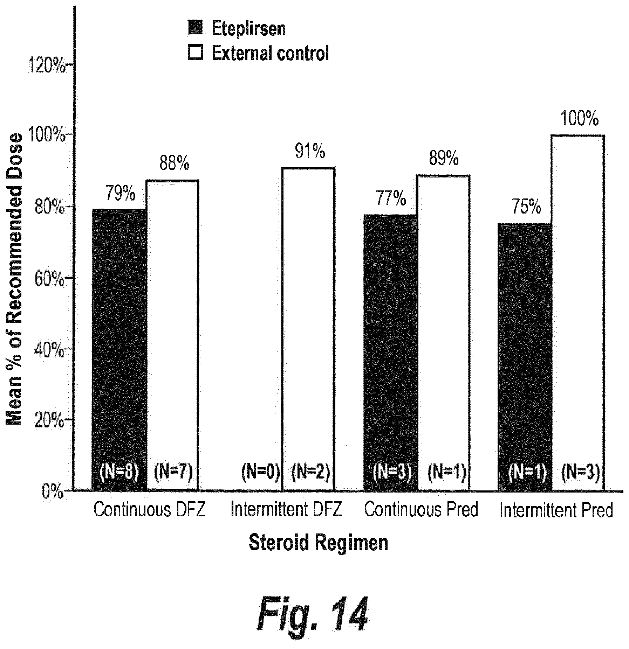

**FIG.14** is a bar graph depicting the mean percentage of recommended dose of steroid (either deflazacort (DFZ) or prednisone (Pred)) administered to patients in each cohort (eteplirsen-treated and the matched external control cohorts).

### DETAILED DESCRIPTION

Embodiments of the present disclosure relate to improved methods for treating muscular dystrophy, such as DMD and BMD, by administering an antisense compound specifically designed to induce exon 51 skipping in the human dystrophin gene, eteplirsen. Dystrophin plays a vital role in muscle function, and various muscle-related diseases are characterized by mutated forms of this gene. Hence, in certain embodiments, the improved methods described herein may be used for inducing exon 51 skipping in mutated forms of the human dystrophin gene, such as the mutated dystrophin genes found in DMD and BMD.

Due to aberrant mRNA splicing events caused by mutations, these mutated human dystrophin genes (i.e., *DMD* gene) either express defective dystrophin protein or express no measurable dystrophin at all, a condition that leads to various forms of muscular dystrophy. To remedy this condition, eteplirsen hybridizes to selected regions of a pre-processed RNA of a mutated human dystrophin gene, induces exon skipping and differential splicing in that otherwise aberrantly spliced dystrophin mRNA, and thereby allows muscle cells to produce an mRNA transcript that encodes a functional dystrophin protein. In certain embodiments, the resulting dystrophin protein is not necessarily the "wild-type" form of dystrophin, but is rather a truncated, yet functional or semi-functional, form of dystrophin.

By increasing the levels of functional dystrophin protein in muscle cells, these and related embodiments are useful in the prophylaxis and treatment of muscular dystrophy, especially those forms of muscular dystrophy, such as DMD and BMD, that are characterized by the expression of defective dystrophin proteins due to aberrant mRNA splicing. The methods described herein further provide improved treatment options for patients with muscular dystrophy and offer significant and practical advantages over alternate methods of treating relevant forms of muscular dystrophy.

Thus, the invention relates to improved methods for treating muscular dystrophy such as DMD and BMD, by inducing exon 51 skipping in a patient. In some embodiments, an effective amount of eteplirsen (e.g., 30 mg per kg of patient body weight) is administered to a DMD patient having a mutation in the DMD gene that is amenable to exon 51 skipping, wherein the administration is over a period of time sufficient to treat the disease. In some embodiments, eteplirsen is administered to the patient at a dose of between about 25 mg/kg and about 50 mg/kg (e.g., about 25, 26, 27, 28, 29, 30, 31, 32, 33, 34, 35, 36, 37, 38, 39, 40, 41, 42, 43, 44, 45, 46, 47, 48, 49, or 50 mg/kg), e.g., once per week. In some embodiments, eteplirsen is administered to the patient at a dose of between about 25 mg/kg and about 50 mg/kg (e.g., about 30 mg/kg to about 50 mg/kg, about 25 mg/kg to about 40 mg/kg, about 28 mg/kg to about 32 mg/kg, or about 30 mg/kg to about 40 mg/kg), e.g., once per week. In some embodiments, an effective amount of eteplirsen is administered to a patient in need thereof for at least 168 (e.g., at least 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, or 216) weeks. In some embodiments, treatment of a DMD patient includes administering eteplirsen (e.g., at a dose of 30 mg/kg) for more than 168 (e.g., more than 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, or more than 216 weeks) weeks. In some embodiments, treatment of a DMD patient includes administering eteplirsen (e.g., at a dose of 30 mg/kg) for at least about 168 to about 192 weeks or about 168 to about 216 weeks. In some embodiments, an effective amount of eteplirsen is administered to a patient in need thereof for at least 192 (e.g., at least 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, or 216) weeks. In some embodiments, an effective amount of eteplirsen is administered to a patient in need thereof for more than 192 (e.g., more than 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 230, 235, 240, 245, 250, 255, or 260 or more) weeks. In some embodiments, treatment of a DMD patient includes administering eteplirsen (e.g., at a dose of 30 mg/kg) for at least 216 (e.g., at least 217, 218, 219, 220, 221, 222, 223, 224, 225, 230, 235, 240, 245, 250, 255, or 260 or more) weeks. In some embodiments, treatment of a DMD patient includes administering eteplirsen (e.g., at a dose of 30 mg/kg) for at least about 192 weeks to about 216 weeks.

Various mutations in the dystrophin gene are amenable to exon 51 skipping. Non-limiting examples of mutations in the following exons are amenable to exon 51 skipping include, e.g.: 45-50, 47-50, 48-50, 49-50, 50, 52, 52-63 (Leiden Duchenne muscular dystrophy mutation database, Leiden University Medical Center, The Netherlands). Determining whether a patient has a mutation in the DMD gene that is amenable to exon skipping is well within the purview of one of skill in the art (see, e.g., Aartsma-Rus et al. (2009) Hum Mut 30:293-299).

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

### I. Definitions

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

The terms "antisense oligomer" and "antisense compound" and "antisense oligonucleotide" are used interchangeably and refer to a sequence of cyclic subunits, each bearing a base-pairing moiety, linked by intersubunit linkages that allow the base-pairing moieties to hybridize to a target sequence in a nucleic acid (typically an RNA) by Watson-Crick base pairing, to form a nucleic acid:oligomer heteroduplex within the target sequence. The cyclic subunits are based on ribose or another pentose sugar or, in a preferred embodiment, a morpholino group (see description of morpholino oligomers below). The oligomer may have exact or near sequence complementarity to the target sequence; variations in sequence near the termini of an oligomer are generally preferable to variations in the interior.

The terms "morpholino oligomer" or "PMO" (phosphoramidate- or phosphorodiamidate morpholino oligomer) refer to an oligonucleotide analog composed of morpholino subunit structures, where (i) the structures are linked together by phosphorus-containing linkages, one to three atoms long, preferably two atoms long, and preferably uncharged or cationic, joining the morpholino nitrogen of one subunit to a 5' exocyclic carbon of an adjacent subunit, and (ii) each morpholino ring bears a purine or pyrimidine base-pairing moiety effective to bind, by base specific hydrogen bonding, to a base in a polynucleotide. See, for example, the structure in Figure 1A, which shows a preferred phosphorodiamidate linkage type. Variations can be made to this linkage as long as they do not interfere with binding or activity. For example, the oxygen attached to phosphorus may be substituted with sulfur (thiophosphorodiamidate). The 5' oxygen may be substituted with amino or lower alkyl substituted amino. The pendant nitrogen attached to phosphorus may be unsubstituted, monosubstituted, or disubstituted with (optionally substituted) lower alkyl. The purine or pyrimidine base pairing moiety is typically adenine, cytosine, guanine, uracil, thymine or inosine. The synthesis, structures, and binding characteristics of morpholino oligomers are detailed in U.S. Patent Nos. 5,698,685, 5,217,866, 5,142,047, 5,034,506, 5,166,315, 5,521,063, 5,506,337, 8,076,476, 8,299,206 and 7,943,762 (cationic linkages), all of which are incorporated herein by reference. Modified intersubunit linkages and terminal groups are detailed in PCT application US2011/038459 and publication WO/2011/150408 which are incorporated herein by reference in their entirety.

"Eteplirsen", also known as "AVN-4658" is a PMO having the base sequence 5'-CTCCAACATCAAGGAAGATGGCATTTCTAG-3' (SEQ ID NO:1). Eteplirsen is registered under CAS Registry Number 1173755-55-9. Chemical names include:
|RNA. [*P-*deoxy-*P*-(dimethylamino)](2',3'-dideoxy-2',3'-imino-2',3'-seco)(2'a→5')(C-m5U-C-C-A-A-C-A-m5U-C-A-A-G-G-A-A-G-A-m5U-G-G-C-A-m5U-m5U-C-5mU-m5U-A-G), 5'-[*P*-[4-[[2-[2-(2-hydroxyethoxy)ethoxy]ethoxy]carbonyl]-1piperazin]-*N*,*N-*dimethylphosphonamidate]
and
*P*,2',3'-trideoxy-*P*-(dimethylamino)-5'-*O*-{*P*-[4-(10-hydroxy-2,5,8-trioxadecanoyl)piperazin-1-yl]-*N*,*N*-dimethylphosphonamidoyl}-2',3'-imino-2',3'-secocytidylyl-(2'a→5')-*P*,3'-dideoxy-*P*-(dimethylamino)-2',3'-imino-2',3'-secothymidylyl-(2'a→5')-*P*, 2',3'-trideoxy-*P*-(dimethylamino)-2',3'-imino-2',3'-secocytidylyl-(2'a→5')-*P*,2',3'-trideoxy-*P*-(dimethylamino)-2',3'-imino-2',3'-secocytidylyl-(2'a→5')-*P*,2',3'-trideoxy-*P*-(dimethylamino)-2',3'-imino-2',3'-secoadenylyl-(2'a→5')-*P*,2',3'-trideoxy-*P-*(dimethylamino)-2',3'-imino-2',3'-secoadenylyl-(2'a→5')-*P*, 2',3'-trideoxy-*P-*(dimethylamino)-2',3'-imino-2',3'-secocytidylyl-(2'a→5')-*P*,2',3'-trideoxy-*P-*(dimethylamino)-2',3'-imino-2',3'-secoadenylyl-(2'a→5')-*P*,3'-dideoxy-*P-*(dimethylamino)-2',3'-imino-2',3'-secothymidylyl-(2'a→5')-*P*,2',3'-trideoxy-*P-*(dimethylamino)-2',3'-imino-2',3'-secocytidylyl-(2'a→5')-*P*,2',3'-trideoxy-*P-*(dimethylamino)-2',3'-imino-2',3'-secoadenylyl-(2'a→5')-*P*,2',3'-trideoxy-*P-*(dimethylamino)-2',3'-imino-2',3'-secoadenylyl-(2'a→5')-*P*,2',3'-trideoxy-*P-*(dimethylamino)-2',3'-imino-2',3'-secoguanylyl-(2'a→5')-*P*,2',3'-trideoxy-*P-*(dimethylamino)-2',3'-imino-2',3'-secoguanylyl-(2'a→5')-*P*,2',3'-trideoxy-*P-*(dimethylamino)-2',3'-imino-2',3'-secoadenylyl-(2'a→5')-*P*,2',3'-trideoxy-*P-*(dimethylamino)-2',3'-imino-2',3'-secoadenylyl-(2'a→5')-*P*,2',3'-trideoxy-*P-*(dimethylamino)-2',3'-imino-2',3'-secoguanylyl-(2'a→5')-*P*,2',3'-trideoxy-*P-*(dimethylamino)-2',3'-imino-2',3'-secoadenylyl-(2'a→5')-*P*,3'-dideoxy-*P-*(dimethylamino)-2',3'-imino-2',3'-secothymidylyl-(2'a→5')-*P*,2',3'-trideoxy-*P-*(dimethylamino)-2',3'-imino-2',3'-secoguanylyl-(2'a→5')-*P*,2',3'-trideoxy-*P-*(dimethylamino)-2',3'-imino-2',3'-secoguanylyl-(2'a→5')-*P*,2',3'-trideoxy-*P-*(dimethylamino)-2',3'-imino-2',3'-secocytidylyl-(2'a→5')-*P*,2',3'-trideoxy-*P-*(dimethylamino)-2',3'-imino-2',3'-secoadenylyl-(2'a→5')-*P*,3'-dideoxy-*P-*(dimethylamino)-2',3'-imino-2',3'-secothymidylyl-(2'a→5')-*P*,3'-dideoxy-*P-*(dimethylamino)-2',3'-imino-2',3'-secothymidylyl-(2'a→5')-*P*,3'-dideoxy-*P-*(dimethylamino)-2',3'-imino-2',3'-secothymidylyl-(2'a→5')-*P*,2',3'-trideoxy-*P-*(dimethy)amino)-2',3'-imino-2',3'-secocytidylyl-(2'a→5')-*P*,3'-dideoxy-*P*-(dimethylamino)-2',3'-imino-2',3'-secothymidylyl-(2'a→5')-*P*,2',3'-trideoxy-*P*-(dimethylamino)-2',3'-imino-2',3'-secoadenylyl-(2'a→5')-2',3'-dideoxy-2',3'-imino-2',3'-secoguanosine
Eteplirsen has the following structure:

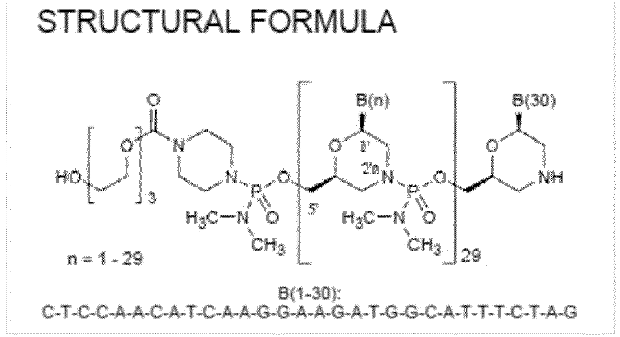

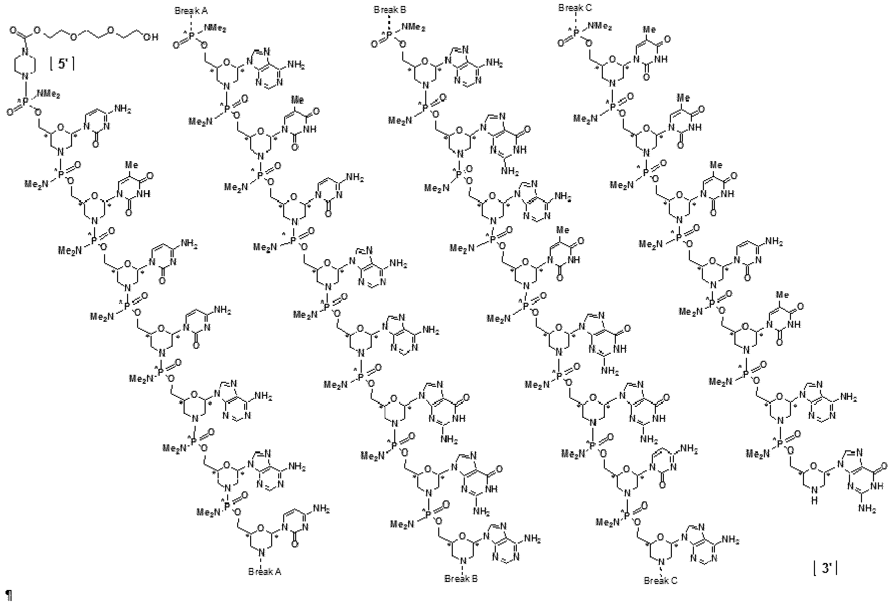

An "exon" refers to a defined section of nucleic acid that encodes for a protein, or a nucleic acid sequence that is represented in the mature form of an RNA molecule after either portions of a pre-processed (or precursor) RNA have been removed by splicing. The mature RNA molecule can be a messenger RNA (mRNA) or a functional form of a non-coding RNA, such as rRNA or tRNA. The human dystrophin gene has about 79 exons.

An "intron" refers to a nucleic acid region (within a gene) that is not translated into a protein. An intron is a non-coding section that is transcribed into a precursor mRNA (pre-mRNA), and subsequently removed by splicing during formation of the mature RNA.

An "effective amount" or "therapeutically effective amount" refers to an amount of therapeutic compound, such as an antisense oligonucleotide, administered to a human subject, either as a single dose or as part of a series of doses, which is effective to produce a desired therapeutic effect. For an antisense oligonucleotide, this effect is typically brought about by inhibiting translation or natural splice-processing of a selected target sequence. In some embodiments, an effective amount is 30 mg/kg of a composition including eteplirsen for a period of time to treat the subject. In some embodiments, an effective amount is about 30 mg/kg of eteplirsen to increase the number of dystrophin-positive fibers in a subject to at least 20% of normal. In another embodiment, an effective amount is about 30 mg/kg of eteplirsen to stabilize, maintain, or improve walking distance from a 20% deficit, for example in a 6 MWT, in a patient, relative to a healthy peer. In another aspect, an effective amount is about 30 mg/kg, once per week for more than 168 weeks (e.g., at least 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, or 216 weeks) to thereby stabilize ambulation (following a loss of ambulation) or improve ambulation (e.g., as measured by the 6MWT) in a patient. In another aspect, an effective amount is about 30 mg/kg once per week for more than 168 weeks (e.g., at least 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, or 216 weeks), such that between about weeks 168 and about 192, patient ambulation is stabilized (following a loss of ambulation) or improved. In another aspect, an effective amount is about 30 mg/kg, once per week for more than 192 weeks to thereby stabilize ambulation (following a loss of ambulation), reduce the rate of loss of ambulation, reduce the loss of ambulation, or improve ambulation (e.g., as measured by the 6MWT) in a patient. In another aspect, an effective amount is about 30 mg/kg once per week for at least 216 weeks, such that between about weeks 192 and about 216, the loss of patient ambulation is reduced, ambulation is stabilized (e.g., relative to baseline), or the rate of loss of ambulation is reduced, relative to matched patients who are not treated with eteplirsen.

"Exon skipping" refers generally to the process by which an entire exon, or a portion thereof, is removed from a given pre-processed RNA, and is thereby excluded from being present in the mature RNA, such as the mature mRNA that is translated into a protein. Hence, the portion of the protein that is otherwise encoded by the skipped exon is not present in the expressed form of the protein, typically creating an altered, though still functional, form of the protein. In certain embodiments, the exon being skipped is an aberrant exon from the human dystrophin gene, which may contain a mutation or other alteration in its sequence that otherwise causes aberrant splicing. In certain embodiments, the exon being skipped is exon 51 of the human dystrophin gene.

"Dystrophin" is a rod-shaped cytoplasmic protein, and a vital part of the protein complex that connects the cytoskeleton of a muscle fiber to the surrounding extracellular matrix through the cell membrane, encoded by the dystrophin (i.e., *DMD*) gene. Dystrophin contains multiple functional domains. For instance, dystrophin contains an actin binding domain at about amino acids 14-240 and a central rod domain at about amino acids 253-3040. This large central domain is formed by 24 spectrin-like triple-helical elements of about 109 amino acids, which have homology to alpha-actinin and spectrin. The repeats are typically interrupted by four proline-rich non-repeat segments, also referred to as hinge regions. Repeats 15 and 16 are separated by an 18 amino acid stretch that appears to provide a major site for proteolytic cleavage of dystrophin. The sequence identity between most repeats ranges from 10-25%. One repeat contains three alpha-helices: 1, 2 and 3. Alpha-helices 1 and 3 are each formed by 7 helix turns, probably interacting as a coiled-coil through a hydrophobic interface. Alpha-helix 2 has a more complex structure and is formed by segments of four and three helix turns, separated by a Glycine or Proline residue. Each repeat is encoded by two exons, typically interrupted by an intron between amino acids 47 and 48 in the first part of alpha-helix 2. The other intron is found at different positions in the repeat, usually scattered over helix-3. Dystrophin also contains a cysteine-rich domain at about amino acids 3080-3360), including a cysteine-rich segment (i.e., 15 Cysteines in 280 amino acids) showing homology to the C-terminal domain of the slime mold (Dictyostelium discoideum) alpha-aclinin. The carboxy-terminal domain is at about amino acids 3361-3685.

The amino-terminus of dystrophin binds to F-actin and the carboxy-terminus binds to the dystrophin-associated protein complex (DAPC) at the sarcolemma. The DAPC includes the dystroglycans, sarcoglycans, integrins and caveolin, and mutations in any of these components cause autosomally inherited muscular dystrophies. The DAPC is destabilized when dystrophin is absent, which results in diminished levels of the member proteins, and in turn leads to progressive fibre damage and membrane leakage. In various forms of muscular dystrophy, such as Duchenne's muscular dystrophy (DMD) and Becker's muscular dystrophy (BMD), muscle cells produce an altered and functionally defective form of dystrophin, or no dystrophin at all, mainly due to mutations in the gene sequence that lead to incorrect splicing. The predominant expression of the defective dystrophin protein, or the complete lack of dystrophin or a dystrophin-like protein, leads to rapid progression of muscle degeneration, as noted above. In this regard, a "defective" dystrophin protein may be characterized by the forms of dystrophin that are produced in certain subjects with DMD or BMD, as known in the art, or by the absence of detectable dystrophin.

As used herein, the terms "function" and "functional" and the like refer to a biological, enzymatic, or therapeutic function.

A "functional" dystrophin protein refers generally to a dystrophin protein having sufficient biological activity to reduce the progressive degradation of muscle tissue that is otherwise characteristic of muscular dystrophy, typically as compared to the altered or "defective" form of dystrophin protein that is present in certain subjects with DMD or BMD. In certain embodiments, a functional dystrophin protein may have about 10%, 20%, 30%, 40%, 50%, 60%, 70%, 80%, 90%, or 100% (including all integers in between) of the in vitro or in vivo biological activity of wild-type dystrophin, as measured according to routine techniques in the art. As one example, dystrophin-related activity in muscle cultures in vitro can be measured according to myotube size, myofibril organization (or disorganization), contractile activity, and spontaneous clustering of acetylcholine receptors (see, e.g., Brown et al., Journal of Cell Science. 112:209-216, 1999). Animal models are also valuable resources for studying the pathogenesis of disease, and provide a means to test dystrophin-related activity. Two of the most widely used animal models for DMD research are the mdx mouse and the golden retriever muscular dystrophy (GRMD) dog, both of which are dystrophin negative (see, e.g., Collins & Morgan, Int J Exp Pathol 84: 165-172, 2003). These and other animal models can be used to measure the functional activity of various dystrophin proteins. Included are truncated forms of dystrophin, such as those forms that are produced by certain of the exon-skipping antisense compounds of the present invention.

The term "restoration" of dystrophin synthesis or production refers generally to the production of a dystrophin protein including truncated forms of dystrophin in a patient with muscular dystrophy following treatment with eteplirsen as described herein. In some embodiments, treatment results in an increase in novel dystrophin production in a patient by 1%, 5%, 10%, 20%, 30%, 40%, 50%, 60%, 70%, 80%, 90% or 100% (including all integers in between). In some embodiments, treatment increases the number of dystrophin-positive fibers to at least 20%, about 30%, about 40%, about 50%, about 60%, about 70%, about 80%, about 90 % or about 95% to 100% of normal in the subject. In other embodiments, treatment increases the number of dystrophin-positive fibers to about 20% to about 60%, or about 30% to about 50% of normal in the subject. The percent of dystrophin-positive fibers in a patient following treatment can be determined by a muscle biopsy using known techniques. For example, a muscle biopsy may be taken from a suitable muscle, such as the biceps brachii muscle in a patient.

Analysis of the percentage of positive dystrophin fibers may be performed pre-treatment and/or post-treatment or at time points throughout the course of treatment. In some embodiments, a post-treatment biopsy is taken from the contralateral muscle from the pre-treatment biopsy. Pre- and post-treatment dystrophin expression studies may be performed using any suitable assay for dystrophin. In one embodiment, immunohistochemical detection is performed on tissue sections from the muscle biopsy using an antibody that is a marker for dystrophin, such as a monoclonal or a polyclonal antibody. For example, the MANDYS106 antibody can be used which is a highly sensitive marker for dystrophin. Any suitable secondary antibody may be used.

In some embodiments, the percent dystrophin-positive fibers are calculated by dividing the number of positive fibers by the total fibers counted. Normal muscle samples have 100% dystrophin-positive fibers. Therefore, the percent dystrophin-positive fibers can be expressed as a percentage of normal. To control for the presence of trace levels of dystrophin in the pretreatment muscle as well as revertant fibers a baseline can be set using sections of pre-treatment muscles from each patient when counting dystrophin-positive fibers in post-treatment muscles. This may be used as a threshold for counting dystrophin-positive fibers in sections of post-treatment muscle in that patient. In other embodiments, antibody-stained tissue sections can also be used for dystrophin quantification using Bioquant image analysis software (Bioquant Image Analysis Corporation, Nashville, TN). The total dystrophin fluorescence signal intensity can be reported as a percentage of normal. In addition, Western blot analysis with monoclonal or polyclonal anti-dystrophin antibodies can be used to determine the percentage of dystrophin positive fibers. For example, the anti-dystrophin antibody NCL-Dys1 from Novacastra may be used. Dystrophin production can also be measured by reverse-transcription polymerase chain reaction (RT-PCR). Primers can be designed to measure dystrophin genes that will produce a functional dystrophin protein. The percentage of dystrophin-positive fibers can also be analyzed by determining the expression of the components of the sarcoglycan complex (β,γ) and/or neuronal NOS.

In some embodiments, treatment with eteplirsen slows or reduces the progressive respiratory muscle dysfunction and/or failure in patients with DMD that would be expected without treatment. In some embodiments, treatment with eteplirsen stabilizes respiratory muscle function in patients with DMD. In one embodiment, treatment with eteplirsen may reduce or eliminate the need for ventilation assistance that would be expected without treatment. In one embodiment, measurements of respiratory function for tracking the course of the disease, as well as the evaluation of potential therapeutic interventions include Maximum inspiratory pressure (MIP), maximum expiratory pressure (MEP) and forced vital capacity (FVC). MIP and MEP measure the level of pressure a person can generate during inhalation and exhalation, respectively, and are sensitive measures of respiratory muscle strength. MIP is a measure of diaphragm muscle weakness.

In one embodiment, MEP may decline before changes in other pulmonary function tests, including MIP and FVC. In another embodiment, MEP may be an early indicator of respiratory dysfunction. In another embodiment, FVC may be used to measure the total volume of air expelled during forced exhalation after maximum inspiration. In patients with DMD, FVC increases concomitantly with physical growth until the early teens. However, as growth slows or is stunted by disease progression, and muscle weakness progresses, the vital capacity enters a descending phase and declines at an average rate of about 8 to 8.5 percent per year after 10 to 12 years of age. In another embodiment, MIP percent predicted (MIP adjusted for weight), MEP percent predicted (MEP adjusted for age) and FVC percent predicted (FVC adjusted for age and height) are supportive analyses.

By "isolated" is meant material that is substantially or essentially free from components that normally accompany it in its native state. For example, an "isolated polynucleotide," as used herein, may refer to a polynucleotide that has been purified or removed from the sequences that flank it in a naturally-occurring state, e.g., a DNA fragment that has been removed from the sequences that are normally adjacent to the fragment.

By "enhance" or "increase" or "restore" or "stimulate," refers generally to the ability of eteplirsen to produce or cause a greater physiological response (i.e., downstream effects) in a cell or a subject, as compared to the response caused by either no eteplirsen or a control compound. A measurable physiological response may include increased expression of a functional form of a dystrophin protein, or increased dystrophin-related biological activity in muscle tissue, among other responses apparent from the understanding in the art and the description herein. Increased muscle function can also be measured, including increases or improvements in muscle function by about 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100%. The percentage of muscle fibers that express a functional dystrophin can also be measured, including increased dystrophin expression in about 1%, 2%, %, 15%, 16%, 17%, 18%, 19%, 20%, 25%, 30%, 35%, 40%, 45%, 50%, 55%, 60%, 65%, 70%, 75%, 80%, 85%, 90%, 95%, or 100% of muscle fibers. For instance, it has been shown that around 40% of muscle function improvement can occur if 25-30% of fibers express dystrophin (see, e.g., DelloRusso et al, Proc Natl Acad Sci USA 99: 12979-12984, 2002). An "increased" or "enhanced" amount may include, e.g., an increase that is 1.1, 1.2, 2, 3, 4, 5, 6, 7, 8, 9, 10, 15, 20, 30, 40, 50 or more times (e.g., 500, 1000 times) (including all integers and decimal points in between and above 1), e.g., 1.5, 1.6, 1.7, 1.8, etc.) the amount produced by no eteplirsen (the absence of an agent) or a control compound.

The term "reduce" or "inhibit" relates generally to the ability of eteplirsen to "decrease" a relevant physiological or cellular response, such as a symptom of a disease or condition described herein, as measured according to routine techniques in the diagnostic art. Relevant physiological or cellular responses (*in vivo* or *in vitro*) will be apparent to persons skilled in the art, and may include reductions in the symptoms or pathology of muscular dystrophy, or reductions in the expression of defective forms of dystrophin, such as the altered forms of dystrophin that are expressed in individuals with DMD or BMD. A "decrease" in a response may include, e.g., a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% decrease, including all integers in between.

"Treatment" of an individual (e.g. a mammal, such as a human) or a cell is any type of intervention used in an attempt to alter the natural course of the individual or cell. Treatment includes, but is not limited to, administration of a pharmaceutical composition, and may be performed either prophylactically or subsequent to the initiation of a pathologic event or contact with an etiologic agent. Treatment includes any desirable effect on the symptoms or pathology of a disease or condition associated with the dystrophin protein, as in certain forms of muscular dystrophy, and may include, for example, minimal changes or improvements in one or more measurable markers of the disease or condition being treated. Also included are "prophylactic" treatments, which can be directed to reducing the rate of progression of the disease or condition being treated, delaying the onset of that disease or condition, or reducing the severity of its onset. "Treatment" or "prophylaxis" does not necessarily indicate complete eradication, cure, or prevention of the disease or condition, or associated symptoms thereof.

In one embodiment, treatment with eteplirsen increases novel dystrophin production and maintains ambulation or slows or reduces the loss of ambulation that would be expected without treatment. For example, treatment may stabilize, maintain, improve or increase walking ability (e.g., stabilization of ambulation) in the subject. In some embodiments, treatment maintains, increases, or reduces loss of a stable walking distance in a patient, as measured by, for example, the 6 Minute Walk Test (6MWT), described by McDonald, et al. (Muscle Nerve, 2010; 42:966-74; Muscle Nerve, 2010; 41:500-10, herein incorporated by reference). The 6MWT is a clinically meaningful endpoint focused on ambulation that characterizes changes in walking function over time as an expression of changes in disease state.

A change in the 6 Minute Walk Distance (6MWD) may be expressed as an absolute value, a percentage change or a change in the %-predicted value. In some embodiments, treatment maintains or improves a stable walking distance in a 6MWT from a 20% deficit in the subject relative to a healthy peer. The performance of a DMD patient in the 6MWT relative to the typical performance of a healthy peer can be determined by calculating a %-predicted value. For example, the %-predicted 6MWD may be calculated using the following equation for males: 196.72 + (39.81 x age) - (1.36 x age²) + (132.28 x height in meters). For females, the %-predicted 6MWD may be calculated using the following equation: 188.61 + (51.50 x age) - (1.86 x age²) + (86.10 x height in meters) (Henricson et al. PLoS Curr., 2012, version 2, herein incorporated by reference). In some embodiments, treatment with eteplirsen increases the stable walking distance in the patient from baseline to greater than 3, 5, 6, 7, 8, 9, 10, 15, 20, 25, 30 or 50 meters (including all integers in between). In some embodiments, treatment with eteplirsen reduces the loss of a stable walking distance. In some embodiments, the patient maintains a 6MWD of at least 50 meters. In some embodiments, the patient maintains a 6MWD of at least 55 meters. In some embodiments, a patient maintains a 6MWD of at least 100, 200, 300 or 400 meters.

Due to decreases in muscle function in patients with DMD, ambulation is often lost during disease progression. Ambulation can be measured through various methods, including the North Star Ambulatory Assessment (NSAA). The NSAA was developed by the Physiotherapy Assessment and Evaluation Group of the North Start Clinical Network to assess ambulant boys with DMD, and provides a list of activities that are scored from 2-0, with 2 being normal and 0 being "unable to achieve independently" (2006-2011 MDC/North Star Clinical Network). These activities range from standing for a minimum of 3 seconds to climbing up and down a box to running. In certain embodiments, treatment with eteplirsen may maintain, stabilize, increase, or improve ambulation, for example, as determined by the NSAA.

In some embodiments, muscle function in a DMD patient may be assessed for ability to rise from a supine position without external assistance (independently). In some embodiments, the assessment can include whether or not the subject has the ability to rise from a supine position. In some embodiments, the assessment includes the time (e.g., in seconds) it takes for the patient to rise from a supine position without external assistance. Such measurements are well known to the skilled artisan and are described in, e.g., Bushby and Connor (2011) Clin Investig (Lond.) 1(9):1217-1235 and Henricson et al. (2013) Muscle Nerve 48(1):55-67, the disclosures of each of which are incorporated herein by reference in their entirety. In some embodiments, a patient treated by any of the methods described herein has lost the ability to rise independently from a supine position prior to beginning treatment with eteplirsen. In some embodiments, the patient lost the ability to rise independently from a supine position at least one (e.g., at least two, three, four, five, six, seven, eight, nine, 10, 11, 12, 16, 18, 20, 24, or more) month(s) prior to beginning treatment with eteplirsen. In some embodiments, a patient treated by any of the methods described herein loses the ability to rise independently from a supine position while being treated with eteplirsen. In some embodiments, the patient loses the ability to rise independently from a supine position at least one (e.g., at least two, three, four, five, six, seven, eight, nine, 10, 11, 12, 16, 18, 20, 24, 30, or 36 or more) month(s) after commencing treatment with eteplirsen.

In some embodiments, a patient treated with eteplirsen suffers no greater than a 60 (e.g., 59, 58, 57, 56, 55, 54, 53, 52, 51, 50, 49, 48, 47, 46, 45, 44, or 43) % reduction in ambulation, relative to baseline (e.g., as measured by the 6MWT), e.g., between about week 168 and about week 192 or between about week 168 and about week 216 of chronic weekly treatment with about 30 mg/kg of eteplirsen. In some embodiments, a patient treated with eteplirsen suffers a loss of no greater than 43 (e.g., 42.5, 42, 41.5, 40, 39.5, 39, 38.5, 38, 37.5, 37, 36.5, or 36) % reduction in ambulation, relative to baseline (e.g., as measured by the 6MWT), e.g., between about week 168 and about week 192 or between about week 168 and about week 216 of chronic weekly treatment with about 30 mg/kg of eteplirsen. In some embodiments, the ambulation of the patient (e.g., the ambulatory ability as measured by the 6MWT or NSAA) is stabilized between about week 168 to about week 192 of treatment with eteplirsen. In some embodiments, the ambulation of the patient is stabilized following about week 168 for at least 25 (e.g., 26, 27, 28, 29, 30, 31, 32, 33, 34, 35, 36, 37, 38, 39, 40, 41, 42, 43, 44, 45, 46, 47, 48, 49, 50, 60, 72, 84, or 96 or more) weeks of chronically weekly treatment with eteplirsen. In some embodiments, the rate of loss of ambulation, or the loss of ambulation of an eteplirsen treated patient, is reduced at about 192 weeks to about 216 weeks. In some embodiments, the rate of loss of ambulation, or the loss of ambulation of an eteplirsen treated patient, is reduced at about 216 weeks relative to baseline.

As used herein, the term "stable disease", "stabilized", "stabilization" or like grammatical terms means a less than 20 (e.g., less than 19, 18, 17, 16, 15, 14, 13, 12, 11, 10, 9, 8, 7, 6, 5, 4, 3, 2, or 1) % increase or decrease is at least one measurable or evaluable aspect of a disease. Thus, in some embodiments, a patient with stable ambulation over a given treatment period has no greater than a 20% decrease in ambulation during that treatment period. In some embodiments, a patient with stable ambulation over a given treatment period has no greater than a 10% decline in ambulation during that treatment period. In some embodiments, a patient with stable ambulation over a given treatment period has no greater than a 5% decline in ambulation during that treatment period. In some embodiments, a patient with stable ambulation over a given treatment period has no greater than a 2.5% decline in ambulation during that treatment period. In some embodiments, a patient with stable ambulation over a given treatment period has no greater than a 1% decline in ambulation during that treatment period. For example, a patient with stable ambulation between about week 168 and about week 192 may be one who experiences an additional loss of ambulation during that period that is no greater than 5 (e.g., less than 4, 3, 2, or 1) %, relative to baseline.

"Once weekly" dosing, dosing "once per week", and "weekly" dosing are used interchangeably herein.

Loss of muscle function in patients with DMD may occur against the background of normal childhood growth and development. Indeed, younger children with DMD may show an increase in distance walked during 6MWT over the course of about 1 year despite progressive muscular impairment. In some embodiments, the 6MWD from patients with DMD is compared to typically developing control subjects and to existing normative data from age and sex matched subjects. In some embodiments, normal growth and development can be accounted for using an age and height based equation fitted to normative data. Such an equation can be used to convert 6MWD to a percent-predicted (%-predicted) value in subjects with DMD. In certain embodiments, analysis of %-predicted 6MWD data represents a method to account for normal growth and development, and may show that gains in function at early ages (e.g., less than or equal to age 7) represent stable rather than improving abilities in patients with DMD (Henricson et al. PLoS Curr., 2012, version 2, herein incorporated by reference).

A "patient," as used herein, includes any person that exhibits a symptom, or is at risk for exhibiting a symptom, which can be treated with eteplirsen, such as a subject that has or is at risk for having DMD or BMD, or any of the symptoms associated with these conditions (e.g., muscle fibre loss).

A "pediatric patient" as used herein is a patient from age 1 to 21, inclusive. In some embodiments, the pediatric patient is a patient from age 7 to 21 (e.g., age 7, 8, 9, 10, 11, 12, 13, 14, 15, 16, 17, 18, 19, 20 or 21). In some embodiments, the pediatric patient is a patient of less than seven years of age. In some embodiments, the pediatric patient is a patient of seven years of age or older.

In some embodiments of any of the methods or compositions described herein, the patient is between about 6 months of age and about 4 years of age, inclusive. For example, in some embodiments, the patient is between at least about 6 (e.g., at least 7, 8, 9, 10, 11, or 12) months of age and less than about 48 (e.g., 47, 46, 45, 44, 43, 42, 41, 40, 39, 38, 37, 36, 35, 34, 33, 32, 31, or 30) months of age. In some embodiments, the patient is at least 6 (e.g., at least 7, 8, 9, 10, 11, 12, 13, 14, 15, 16, 17, 18, 19, 20, 24, 30, or 36) months of age. In some embodiments, the patient is no greater than 4 years of age. In some embodiments, eteplirsen may be administered to such patients parenterally, e.g., subcutaneously.

### II. Formulations and Treatment

In certain embodiments, the present invention provides formulations or compositions suitable for the therapeutic delivery of eteplirsen, as described herein. Hence, in certain embodiments, the present invention provides pharmaceutically acceptable compositions that comprise a therapeutically-effective amount of eteplirsen, formulated together with one or more pharmaceutically acceptable carriers (additives) and/or diluents. While it is possible for eteplirsen to be administered alone, it is preferable to administer the compound as a pharmaceutical formulation (composition).

The compositions comprising eteplirsen of the present invention may be administered alone or in combination with another therapeutic. The additional therapeutic may be administered prior, concurrently or subsequently to the administration of the composition of the present invention. For example, the compositions may be administered in combination with a steroid and/or an antibiotic. In certain embodiments, the compositions are administered to a patient that is on background steroid therapy (e.g., intermittent or chronic/continuous background steroid therapy). One of skill in the art would appreciate that such patients are those who are subject to ongoing, chronic use of steroids (or corticosteroids) on top of which another treatment, such as eteplirsen, is administered. For example, in some embodiments the patient has been treated with a corticosteroid (e.g., a stable dose of a corticosteroid for four to six, seven, eight, nine, 10, 11, 12, 13, 14, 15, 16, 17, 18, 19, 20, 21, 22, 23, or 24 or more weeks) prior to administration of eteplirsen and continues to receive the steroid therapy. The steroid may be a glucocorticoid or prednisone. Glucocorticoids such as cortisol control carbohydrate, fat and protein metabolism, and are anti-inflammatory by preventing phospholipid release, decreasing eosinophil action and a number of other mechanisms. Mineralocorticoids such as aldosterone control electrolyte and water levels, mainly by promoting sodium retention in the kidney. Corticosteroids are a class of chemicals that includes steroid hormones naturally produced in the adrenal cortex of vertebrates and analogues of these hormones that are synthesized in laboratories. Corticosteroids are involved in a wide range of physiological processes, including stress response, immune response, and regulation of inflammation, carbohydrate metabolism, protein catabolism, blood electrolyte levels, and behavior. Corticosteroids include, but are not limited to, Betamethasone, Budesonide, Cortisone. Dexamethasone, Hydrocortisone, Methylprednisolone, Prednisolone, and Prednisone. One particular steroid of interest that may be administered prior, concurrently or subsequently to the administration of the composition of the present invention is deflazacort and formulations thereof (e.g., MP-104, Marathon Pharmaceuticals LLC).

In some embodiments, treatment of patients with eteplirsen may lower the amount of a steroid co-therapy required to maintain a similar level, the same, or even better efficacy than that achieved on a higher dose of the steroid and in the absence of eteplirsen. In some embodiments, patients may be administered dosages of a steroid, such as deflazacort or prednisone, that is at least 5 (e.g., at least 6, 7, 8, 9, 10, 11, 12, 13, 14, 15, 16, 17, 18, 19, 20, 21, 22, 23, 24, 25, 26, 27, 28, 29, 30, 32, 35, 37, 40, 45, 50, 55, 60, 65, or 70) % less than the recommended dose (e.g., as recommended by the CDC/TREAT-NMD guidelines; see, Bushby K, Lynn S, Straub V. Collaborating to bring new therapies to the patient: the TREAT-NMD model. Acta Myo 2009;28:12-15) of steroid for a patient of similar level of disease state or progression. In some embodiments, eteplirsen-treated patients are administered between about 75% to about 80% of the recommended dose of a given steroid.

According to the guidelines, the recommended starting dose of prednisone is 0.75 mg/kg/day and that of deflazacort is 0.9 mg/kg/day, given in the morning. Some children experience short-lived behavioral side effects (hyperactivity, mood swings) for a few hours after the medication is given. For these children, administration of the medication in the afternoon may alleviate some of these difficulties. For ambulatory individuals, the dosage is commonly increased as the child grows until he reaches approximately 40 kg in weight. The maximum dose of prednisone is usually capped at approximately 30 mg/day, and that of deflazacort at 36 mg/day. Non-ambulatory teenagers maintained on long-term steroid therapy are usually above 40 kg in weight and the prednisone dosage per kg is often allowed to drift down to the 0.3 to 0.6 mg/kg/day range. While this dosage is less than the approximate 30 mg cap, it demonstrates substantial benefit.

Deciding on a maintenance dose of steroid is a balance between growth of the patient, patient response to steroid therapy, and the burden of side effects. This decision needs to be reviewed at every clinic visit based on the result of the tests done and whether or not side effects are a problem that cannot be managed or tolerated. In DMD patients on a relatively low dosage of steroid (less than the starting dose per kg body weight) who start to show functional decline, it may be necessary to consider a "functional rescue" adjustment. In this situation, the dosage of steroid is increased to the target and the patient is then reevaluated for any benefit in approximately two to three months.

Other agents which can be administered include an antagonist of the ryanodine receptor, such as dantrolene, which has been shown to enhance antisense-mediated exon skipping in patient cells and a mouse model of DMD (G. Kendall et al. Sci Tranl Med 4:164-160 (2012), incorporated herein by reference).

Methods for the delivery of nucleic acid molecules are described, for example, in Akhtar et al., 1992, Trends Cell Bio., 2:139; and Delivery Strategies for Antisense Oligonucleotide Therapeutics, ed. Akhtar; Sullivan et al., PCT WO 94/02595. These and other protocols can be utilized for the delivery of virtually any nucleic acid molecule, including eteplirsen.

As detailed below, the pharmaceutical compositions of the present invention may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient.

Some examples of materials that can serve as pharmaceutically-acceptable carriers include, without limitation: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

Additional non-limiting examples of agents suitable for formulation with eteplirsen include: PEG conjugated nucleic acids, phospholipid conjugated nucleic acids, nucleic acids containing lipophilic moieties, phosphorothioates, P-glycoprotein inhibitors (such as Pluronic P85) which can enhance entry of drugs into various tissues; biodegradable polymers, such as poly (DL-lactide-coglycolide) microspheres for sustained release delivery after implantation (Emerich, D F et al., 1999, Cell Transplant, 8, 47-58) Alkermes, Inc. Cambridge, Mass.; and loaded nanoparticles, such as those made of polybutylcyanoacrylate, which can deliver drugs across the blood brain barrier and can alter neuronal uptake mechanisms (Prog Neuropsychopharmacol Biol Psychiatry, 23, 941-949, 1999).

The invention also features the use of the composition comprising surface-modified liposomes containing poly (ethylene glycol) lipids (PEG-modified, branched and unbranched or combinations thereof, or long-circulating liposomes or stealth liposomes). Eteplirsen can also comprise covalently attached PEG molecules of various molecular weights. These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al. Chem. Rev. 1995, 95, 2601-2627; Ishiwata et al., Chem. Pharm. Bull. 1995, 43, 1005-1011). Such liposomes have been shown to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes which are known to accumulate in tissues of the MPS (Liu et al., J. Biol. Chem. 1995, 42, 24864-24870; Choi et al., International PCT Publication No. WO 96/10391; Ansell et al., International PCT Publication No. WO 96/10390; Holland et al., International PCT Publication No. WO 96/10392). Long-circulating liposomes are also likely to protect drugs from nuclease degradation to a greater extent compared to cationic liposomes, based on their ability to avoid accumulation in metabolically aggressive MPS tissues such as the liver and spleen.

In a further embodiment, the present invention includes eteplirsen prepared for delivery as described in U.S. Pat. Nos. 6,692,911, 7,163,695 and 7,070,807. In this regard, in one embodiment, the present invention provides eteplirsen in a composition comprising copolymers of lysine and histidine (HK) (as described in U.S. Pat. Nos. 7,163,695, 7,070,807, and 6,692,911) either alone or in combination with PEG (e.g., branched or unbranched PEG or a mixture of both), in combination with PEG and a targeting moiety or any of the foregoing in combination with a crosslinking agent. In certain embodiments, the present invention provides eteplirsen in a composition comprising gluconic-acid-modified polyhistidine or gluconylated-polyhistidine/transferrin-polylysine. One skilled in the art will also recognize that amino acids with properties similar to His and Lys may be substituted within the composition.

Certain embodiments of eteplirsen may contain a basic functional group, such as amino or alkylamino, and are, thus, capable of forming pharmaceutically-acceptable salts with pharmaceutically-acceptable acids. The term "pharmaceutically-acceptable salts" in this respect, refers to the relatively non-toxic, inorganic and organic acid addition salts of compounds of the present invention. These salts can be prepared in situ in the administration vehicle or the dosage form manufacturing process, or by separately reacting a purified compound of the invention in its free base form with a suitable organic or inorganic acid, and isolating the salt thus formed during subsequent purification. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, e.g., Berge et al. (1977) "Pharmaceutical Salts", J. Pharm. Sci. 66:1-19).

The pharmaceutically acceptable salts of eteplirsen include the conventional nontoxic salts or quaternary ammonium salts of the compounds, e.g., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In certain embodiments, eteplirsen may contain one or more acidic functional groups and, thus, is capable of forming pharmaceutically-acceptable salts with pharmaceutically-acceptable bases. The term "pharmaceutically-acceptable salts" in these instances refers to the relatively non-toxic, inorganic and organic base addition salts of compounds of the present invention. These salts can likewise be prepared in situ in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, e.g., Berge et al., *supra*).

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient that can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 0.1 percent to about ninety-nine percent of active ingredient, preferably from about 5 percent to about 70 percent, most preferably from about 10 percent to about 30 percent.

In certain embodiments, a formulation of the present invention comprises an excipient selected from cyclodextrins, celluloses, liposomes, micelle forming agents, e.g., bile acids, and polymeric carriers, e.g., polyesters and polyanhydrides; and eteplirsen. In certain embodiments, an aforementioned formulation renders orally bioavailable eteplirsen.

Methods of preparing these formulations or compositions include the step of bringing into association eteplirsen with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. Eteplirsen may also be administered as a bolus, electuary or paste.

In solid dosage forms of the invention for oral administration (capsules, tablets, pills, dragees, powders, granules, trouches and the like), the active ingredient may be mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (e.g., gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations or dosage forms for the topical or transdermal administration of an oligomer as provided herein include powders, sprays, ointments, pastes, creams, lotions, gels, solutions, patches and inhalants. Eteplirsen may be mixed under sterile conditions with a pharmaceutically-acceptable carrier, and with any preservatives, buffers, or propellants which may be required. The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to eteplirsen, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of an oligomer of the present invention to the body. Such dosage forms can be made by dissolving or dispersing the oligomer in the proper medium. Absorption enhancers can also be used to increase the flux of the agent across the skin. The rate of such flux can be controlled by either providing a rate controlling membrane or dispersing the agent in a polymer matrix or gel, among other methods known in the art.

Pharmaceutical compositions suitable for parenteral administration may comprise eteplirsen n in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon eteplirsen may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility, among other methods known in the art. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms may be made by forming microencapsule matrices of eteplirsen in biodegradable polymers such as polylactide-polyglycolide. Depending on the ratio of eteplirsen to polymer, and the nature of the particular polymer employed, the rate of eteplirsen release can be controlled. Examples of other biodegradable polymers include poly(orthoesters) and poly(anhydrides). Depot injectable formulations may also prepared by entrapping the drug in liposomes or microemulsions that are compatible with body tissues.

When eteplirsen is administered as a pharmaceutical, to humans and animals, it can be given per se or as a pharmaceutical composition containing, for example, 0.1 to 99% (more preferably, 10 to 30%) of active ingredient in combination with a pharmaceutically acceptable carrier.

As noted above, the formulations or preparations of the present invention may be given orally, parenterally, systemically, topically, rectally or intramuscular administration. They are typically given in forms suitable for each administration route. For example, they are administered in tablets or capsule form, by injection, inhalation, eye lotion, ointment, suppository, etc. administration by injection, infusion or inhalation; topical by lotion or ointment; and rectal by suppositories.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

Regardless of the route of administration selected, eteplirsen, which may be used in a suitable hydrated form, and/or the pharmaceutical compositions of the present invention, may be formulated into pharmaceutically-acceptable dosage forms by conventional methods known to those of skill in the art. Actual dosage levels of the active ingredients in the pharmaceutical compositions of this invention may be varied so as to obtain an amount of the active ingredient which is effective to achieve the desired therapeutic response for a particular patient, composition, and mode of administration, without being unacceptably toxic to the patient.

The selected dosage level will depend upon a variety of factors including the activity of eteplirsen, or the ester, salt or amide thereof, the route of administration, the time of administration, the rate of excretion or metabolism of eteplirsen, the rate and extent of absorption, the duration of the treatment, other drugs, compounds and/or materials used in combination with eteplirsen, the age, sex, weight, condition, general health and prior medical history of the patient being treated, and like factors well known in the medical arts.

In some embodiments, the dosage of eteplirsen is about 30 mg/kg over a period of time sufficient to treat DMD or BMD. In some embodiments, eteplirsen is administered to the patient at a dose of between about 25 mg/kg and about 50 mg/kg (e.g., about 25, 26, 27, 28, 29, 30, 31, 32, 33, 34, 35, 36, 37, 38, 39, 40, 41, 42, 43, 44, 45, 46, 47, 48, 49, or 50 mg/kg), e.g., once per week. In some embodiments, eteplirsen is administered to the patient at a dose of between about 25 mg/kg and about 50 mg/kg (e.g., about 30 mg/kg to about 50 mg/kg, about 25 mg/kg to about 40 mg/kg, about 28 mg/kg to about 32 mg/kg, or about 30 mg/kg to about 40 mg/kg), e.g., once per week.

In some embodiments, eteplirsen is administered intravenously once a week. In certain embodiments, the time of infusion is from about 15 minutes to about 4 hours. In some embodiments, the time of infusion is from about 30 minutes to about 3 hours. In some embodiments, the time of infusion is from about 30 minutes to about 2 hours. In some embodiments, the time of infusion is from about 1 hour to about 2 hours. In some embodiments the time of infusion is from about 30 minutes to about 1 hour. In some embodiments, the time of infusion is about 60 minutes. In some embodiments, the time of infusion is 35 to 60 minutes.

Administration of eteplirsen may be followed by, or concurrent with, administration of an antibiotic, steroid or other therapeutic agent. The treatment regimen may be adjusted (dose, frequency, route, etc.) as indicated, based on the results of immunoassays, other biochemical tests and physiological examination of the subject under treatment.

Nucleic acid molecules can be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres, as described herein and known in the art. In certain embodiments, microemulsification technology may be utilized to improve bioavailability of lipophilic (water insoluble) pharmaceutical agents. Examples include Trimetrine (Dordunoo, S. K., et al., Drug Development and Industrial Pharmacy, 17(12), 1685-1713, 1991 and REV 5901 (Sheen, P. C., et al., J Pharm Sci 80(7), 712-714, 1991). Among other benefits, microemulsification provides enhanced bioavailability by preferentially directing absorption to the lymphatic system instead of the circulatory system, which thereby bypasses the liver, and prevents destruction of the compounds in the hepatobiliary circulation.

In one aspect of invention, the formulations contain micelles formed from eteplirsen and at least one amphiphilic carrier, in which the micelles have an average diameter of less than about 100 nm. More preferred embodiments provide micelles having an average diameter less than about 50 nm, and even more preferred embodiments provide micelles having an average diameter less than about 30 nm, or even less than about 20 nm.

While all suitable amphiphilic carriers are contemplated, the presently preferred carriers are generally those that have Generally-Recognized-as-Safe (GRAS) status, and that can both solubilize the compound of the present invention and microemulsify it at a later stage when the solution comes into a contact with a complex water phase (such as one found in human gastrointestinal tract). Usually, amphiphilic ingredients that satisfy these requirements have HLB (hydrophilic to lipophilic balance) values of 2-20, and their structures contain straight chain aliphatic radicals in the range of C-6 to C-20. Examples are polyethylene-glycolized fatty glycerides and polyethylene glycols.

Examples of amphiphilic carriers include saturated and monounsaturated polyethyleneglycolyzed fatty acid glycerides, such as those obtained from fully or partially hydrogenated various vegetable oils. Such oils may advantageously consist of tri-, di-, and mono-fatty acid glycerides and di- and mono-polyethyleneglycol esters of the corresponding fatty acids, with a particularly preferred fatty acid composition including capric acid 4-10, capric acid 3-9, lauric acid 40-50, myristic acid 14-24, palmitic acid 4-14 and stearic acid 5-15%. Another useful class of amphiphilic carriers includes partially esterified sorbitan and/or sorbitol, with saturated or mono-unsaturated fatty acids (SPAN-series) or corresponding ethoxylated analogs (TWEEN-series).

Commercially available amphiphilic carriers may be particularly useful, including Gelucire-series, Labrafil, Labrasol, or Lauroglycol (all manufactured and distributed by Gattefosse Corporation, Saint Priest, France), PEG-mono-oleate, PEG-di-oleate, PEG-monolaurate and di-laurate, Lecithin, Polysorbate 80, etc (produced and distributed by a number of companies in USA and worldwide).

In certain embodiments, the delivery may occur by use of liposomes, nanocapsules, microparticles, microspheres, lipid particles, vesicles, and the like, for the introduction of the compositions of the present invention into suitable host cells. In particular, the compositions of the present invention may be formulated for delivery either encapsulated in a lipid particle, a liposome, a vesicle, a nanosphere, a nanoparticle or the like. The formulation and use of such delivery vehicles can be carried out using known and conventional techniques.

Hydrophilic polymers suitable for use in the present invention are those which are readily water-soluble, can be covalently attached to a vesicle-forming lipid, and which are tolerated in vivo without toxic effects (i.e., are biocompatible). Suitable polymers include polyethylene glycol (PEG), polylactic (also termed polylactide), polyglycolic acid (also termed polyglycolide), a polylactic-polyglycolic acid copolymer, and polyvinyl alcohol. In certain embodiments, polymers have a molecular weight of from about 100 or 120 daltons up to about 5,000 or 10,000 daltons, or from about 300 daltons to about 5,000 daltons. In other embodiments, the polymer is polyethyleneglycol having a molecular weight of from about 100 to about 5,000 daltons, or having a molecular weight of from about 300 to about 5,000 daltons. In certain embodiments, the polymer is polyethyleneglycol of 750 daltons (PEG(750)). Polymers may also be defined by the number of monomers therein; a preferred embodiment of the present invention utilizes polymers of at least about three monomers, such PEG polymers consisting of three monomers (approximately 150 daltons).

Other hydrophilic polymers which may be suitable for use in the present invention include polyvinylpyrrolidone, polymethoxazoline, polyethyloxazoline, polyhydroxypropyl methacrylamide, polymethacrylamide, polydimethylacrylamide, and derivatized celluloses such as hydroxymethylcellulose or hydroxyethylcellulose.

In certain embodiments, a formulation of the present invention comprises a biocompatible polymer selected from the group consisting of polyamides, polycarbonates, polyalkylenes, polymers of acrylic and methacrylic esters, polyvinyl polymers, polyglycolides, polysiloxanes, polyurethanes and co-polymers thereof, celluloses, polypropylene, polyethylenes, polystyrene, polymers of lactic acid and glycolic acid, polyanhydrides, poly(ortho)esters, poly(butic acid), poly(valeric acid), poly(lactide-co-caprolactone), polysaccharides, proteins, polyhyaluronic acids, polycyanoacrylates, and blends, mixtures, or copolymers thereof.

Cyclodextrins are cyclic oligosaccharides, consisting of 6, 7 or 8 glucose units, designated by the Greek letter α, β, or γ, respectively. The glucose units are linked by α-1,4-glucosidic bonds. As a consequence of the chair conformation of the sugar units, all secondary hydroxyl groups (at C-2, C-3) are located on one side of the ring, while all the primary hydroxyl groups at C-6 are situated on the other side. As a result, the external faces are hydrophilic, making the cyclodextrins water-soluble. In contrast, the cavities of the cyclodextrins are hydrophobic, since they are lined by the hydrogen of atoms C-3 and C-5, and by ether-like oxygens. These matrices allow complexation with a variety of relatively hydrophobic compounds, including, for instance, steroid compounds such as 17α-estradiol (see, e.g., van Uden et al. Plant Cell Tiss. Org. Cult. 38:1-3-113 (1994)). The complexation takes place by Van der Waals interactions and by hydrogen bond formation. For a general review of the chemistry of cyclodextrins, see, Wenz, Agnew. Chem. Int. Ed. Engl., 33:803-822 (1994).

The physico-chemical properties of the cyclodextrin derivatives depend strongly on the kind and the degree of substitution. For example, their solubility in water ranges from insoluble (e.g., triacetyl-beta-cyclodextrin) to 147% soluble (w/v) (G-2-beta-cyclodextrin). In addition, they are soluble in many organic solvents. The properties of the cyclodextrins enable the control over solubility of various formulation components by increasing or decreasing their solubility.

Numerous cyclodextrins and methods for their preparation have been described. For example, Parmeter (I), et al. (U.S. Pat. No. 3,453,259) and Gramera, et al. (U.S. Pat. No. 3,459,731) described electroneutral cyclodextrins. Other derivatives include cyclodextrins with cationic properties [Parmeter (II), U.S. Pat. No. 3,453,257], insoluble crosslinked cyclodextrins (Solms, U.S. Pat. No. 3,420,788), and cyclodextrins with anionic properties [Parmeter (III), U.S. Pat. No. 3,426,011]. Among the cyclodextrin derivatives with anionic properties, carboxylic acids, phosphorous acids, phosphinous acids, phosphonic acids, phosphoric acids, thiophosphonic acids, thiosulphinic acids, and sulfonic acids have been appended to the parent cyclodextrin [see, Parmeter (III), supra]. Furthermore, sulfoalkyl ether cyclodextrin derivatives have been described by Stella, et al. (U.S. Pat. No. 5,134,127).

Liposomes consist of at least one lipid bilayer membrane enclosing an aqueous internal compartment. Liposomes may be characterized by membrane type and by size. Small unilamellar vesicles (SUVs) have a single membrane and typically range between 0.02 and 0.05 µm in diameter; large unilamellar vesicles (LUVS) are typically larger than 0.05 µm. Oligolamellar large vesicles and multilamellar vesicles have multiple, usually concentric, membrane layers and are typically larger than 0.1 µm. Liposomes with several nonconcentric membranes, i.e., several smaller vesicles contained within a larger vesicle, are termed multivesicular vesicles.

One aspect of the present invention relates to formulations comprising liposomes containing eteplirsen, where the liposome membrane is formulated to provide a liposome with increased carrying capacity. Alternatively or in addition, the compound of the present invention may be contained within, or adsorbed onto, the liposome bilayer of the liposome. Eteplirsen may be aggregated with a lipid surfactant and carried within the liposome's internal space; in these cases, the liposome membrane is formulated to resist the disruptive effects of the active agent-surfactant aggregate.

According to one embodiment of the present invention, the lipid bilayer of a liposome contains lipids derivatized with polyethylene glycol (PEG), such that the PEG chains extend from the inner surface of the lipid bilayer into the interior space encapsulated by the liposome, and extend from the exterior of the lipid bilayer into the surrounding environment.

Active agents contained within liposomes of the present invention are in solubilized form. Aggregates of surfactant and active agent (such as emulsions or micelles containing the active agent of interest) may be entrapped within the interior space of liposomes according to the present invention. A surfactant acts to disperse and solubilize the active agent, and may be selected from any suitable aliphatic, cycloaliphatic or aromatic surfactant, including but not limited to biocompatible lysophosphatidylcholines (LPGs) of varying chain lengths (for example, from about C14 to about C20). Polymer-derivatized lipids such as PEG-lipids may also be utilized for micelle formation as they will act to inhibit micelle/membrane fusion, and as the addition of a polymer to surfactant molecules decreases the CMC of the surfactant and aids in micelle formation. Preferred are surfactants with CMOs in the micromolar range; higher CMC surfactants may be utilized to prepare micelles entrapped within liposomes of the present invention.

Liposomes according to the present invention may be prepared by any of a variety of techniques that are known in the art. See, e.g., U.S. Pat. No. 4,235,871; Published PCT applications WO 96/14057; New RRC, Liposomes: A practical approach, IRL Press, Oxford (1990), pages 33-104; Lasic DD, Liposomes from physics to applications, Elsevier Science Publishers BV, Amsterdam, 1993. For example, liposomes of the present invention may be prepared by diffusing a lipid derivatized with a hydrophilic polymer into preformed liposomes, such as by exposing preformed liposomes to micelles composed of lipid-grafted polymers, at lipid concentrations corresponding to the final mole percent of derivatized lipid which is desired in the liposome. Liposomes containing a hydrophilic polymer can also be formed by homogenization, lipid-field hydration, or extrusion techniques, as are known in the art.

In another exemplary formulation procedure, the active agent is first dispersed by sonication in a lysophosphatidylcholine or other low CMC surfactant (including polymer grafted lipids) that readily solubilizes hydrophobic molecules. The resulting micellar suspension of active agent is then used to rehydrate a dried lipid sample that contains a suitable mole percent of polymer-grafted lipid, or cholesterol. The lipid and active agent suspension is then formed into liposomes using extrusion techniques as are known in the art, and the resulting liposomes separated from the unencapsulated solution by standard column separation.

In one aspect of the present invention, the liposomes are prepared to have substantially homogeneous sizes in a selected size range. One effective sizing method involves extruding an aqueous suspension of the liposomes through a series of polycarbonate membranes having a selected uniform pore size; the pore size of the membrane will correspond roughly with the largest sizes of liposomes produced by extrusion through that membrane. See e.g., U.S. Pat. No. 4,737,323 (Apr. 12, 1988). In certain embodiments, reagents such as DharmaFECT® and Lipofectamine® may be utilized to introduce polynucleotides or proteins into cells.

The release characteristics of a formulation of the present invention depend on the encapsulating material, the concentration of encapsulated drug, and the presence of release modifiers. For example, release can be manipulated to be pH dependent, for example, using a pH sensitive coating that releases only at a low pH, as in the stomach, or a higher pH, as in the intestine. An enteric coating can be used to prevent release from occurring until after passage through the stomach. Multiple coatings or mixtures of cyanamide encapsulated in different materials can be used to obtain an initial release in the stomach, followed by later release in the intestine. Release can also be manipulated by inclusion of salts or pore forming agents, which can increase water uptake or release of drug by diffusion from the capsule. Excipients which modify the solubility of the drug can also be used to control the release rate. Agents which enhance degradation of the matrix or release from the matrix can also be incorporated. They can be added to the drug, added as a separate phase (i.e., as particulates), or can be co-dissolved in the polymer phase depending on the compound. In most cases the amount should be between 0.1 and thirty percent (w/w polymer). Types of degradation enhancers include inorganic salts such as ammonium sulfate and ammonium chloride, organic acids such as citric acid, benzoic acid, and ascorbic acid, inorganic bases such as sodium carbonate, potassium carbonate, calcium carbonate, zinc carbonate, and zinc hydroxide, and organic bases such as protamine sulfate, spermine, choline, ethanolamine, diethanolamine, and triethanolamine and surfactants such as Tween® and Pluronic®. Pore forming agents which add microstructure to the matrices (i.e., water soluble compounds such as inorganic salts and sugars) are added as particulates. The range is typically between one and thirty percent (w/w polymer).

Uptake can also be manipulated by altering residence time of the particles in the gut. This can be achieved, for example, by coating the particle with, or selecting as the encapsulating material, a mucosal adhesive polymer. Examples include most polymers with free carboxyl groups, such as chitosan, celluloses, and especially polyacrylates (as used herein, polyacrylates refers to polymers including acrylate groups and modified acrylate groups such as cyanoacrylates and methacrylates).

Eteplirsen may be formulated to be contained within, or, adapted to release by a surgical or medical device or implant. In certain aspects, an implant may be coated or otherwise treated with eteplirsen. For example, hydrogels, or other polymers, such as biocompatible and/or biodegradable polymers, may be used to coat an implant with the compositions of the present invention (i.e., the composition may be adapted for use with a medical device by using a hydrogel or other polymer). Polymers and copolymers for coating medical devices with an agent are well-known in the art. Examples of implants include, but are not limited to, stents, drug-eluting stents, sutures, prosthesis, vascular catheters, dialysis catheters, vascular grafts, prosthetic heart valves, cardiac pacemakers, implantable cardioverter defibrillators, IV needles, devices for bone setting and formation, such as pins, screws, plates, and other devices, and artificial tissue matrices for wound healing.

In addition to the methods provided herein, eteplirsen may be formulated for administration in any convenient way for use in human or veterinary medicine, by analogy with other pharmaceuticals. Eteplirsen and its corresponding formulation may be administered alone or in combination with other therapeutic strategies in the treatment of muscular dystrophy, such as myoblast transplantation, stem cell therapies, administration of aminoglycoside antibiotics, proteasome inhibitors, and up-regulation therapies (e.g., upregulation of utrophin, an autosomal paralogue of dystrophin).

The routes of administration described are intended only as a guide since a skilled practitioner will be able to determine readily the optimum route of administration and any dosage for any particular animal and condition. Multiple approaches for introducing functional new genetic material into cells, both in vitro and in vivo have been attempted (Friedmann (1989) Science, 244:1275-1280). These approaches include integration of the gene to be expressed into modified retroviruses (Friedmann (1989) supra; Rosenberg (1991) Cancer Research 51(18), suppl.: 5074S-5079S); integration into non-retrovirus vectors (e.g., adeno-associated viral vectors) (Rosenfeld, et al. (1992) Cell, 68:143-155; Rosenfeld, et al. (1991) Science, 252:431-434); or delivery of a transgene linked to a heterologous promoter-enhancer element via liposomes (Friedmann (1989), supra; Brigham, et al. (1989) Am. J. Med. Sci., 298:278-281; Nabel, et al. (1990) Science, 249:1285-1288; Hazinski, et al. (1991) Am. J. Resp. Cell Molec. Biol., 4:206-209; and Wang and Huang (1987) Proc. Natl. Acad. Sci. (USA), 84:7851-7855); coupled to ligand-specific, cation-based transport systems (Wu and Wu (1988) J. Biol. Chem., 263:14621-14624) or the use of naked DNA, expression vectors (Nabel et al. (1990), supra); Wolff et al. (1990) Science, 247:1465-1468). Direct injection of transgenes into tissue produces only localized expression (Rosenfeld (1992) supra); Rosenfeld et al. (1991) supra; Brigham et al. (1989) supra; Nabel (1990) supra; and Hazinski et al. (1991) supra). The Brigham et al. group (Am. J. Med. Sci. (1989) 298:278-281 and Clinical Research (1991) 39 (abstract)) have reported in vivo transfection only of lungs of mice following either intravenous or intratracheal administration of a DNA liposome complex. An example of a review article of human gene therapy procedures is: Anderson, Science (1992) 256:808-813.

### III. Kits

The invention also provides kits for treatment of a patient with a genetic disease which kit comprises at least eteplirsen, packaged in a suitable container, together with instructions for its use. The kits may also contain peripheral reagents such as buffers, stabilizers, etc.

### IV. EXAMPLES

### Materials and Methods

### Patients

For the eteplirsen treated cohort, eligible patients were between 7 and 13 years of age (inclusive), with out-of-frame deletions of the DMD gene that could be corrected by skipping exon 51. Patients were confirmed to have stable cardiac and pulmonary function and a stable dose of glucocorticoids for at least 24 weeks prior to enrollment. Only patients who could walk between 200 and 400 meters (±10%) on the 6-Minute Walk Test (6MWT) at baseline were enrolled.

### Study Design

### Eteplirsen-Treated Cohort

This 4 year trial was conducted in two phases: (1) treatment was double-blind through week 24 and (2) open-label thereafter. The primary endpoint was change in ambulation as measured by the 6-Minute Walk Test (6MWT).

Study 201 (Study no. 4658-us-201; ClinicalTrials.gov Identifier: NCT01396239) was a single-site, randomized, double-blind, placebo-controlled, multiple-dose efficacy, safety and tolerability trial of eteplirscn. Twelve patients with DMD were randomized to one of three groups: eteplirsen 30 mg/kg/week (Cohort 1); eteplirsen 50 mg/kg/week (Cohort 2); or placebo/delayed eteplirsen (Cohort 3). All patients received weekly intravenous eteplirsen or placebo/delayed eteplirsen dosing. Placebo-treated patients crossed over to weekly eteplirsen 30 (n=2) or 50 mg/kg (n=2) at week 25. Efficacy and safety were assessed at scheduled visits, and an independent Data Safety Monitoring Board ensured the welfare of all patients. All patients had bicep biopsies at baseline. Follow-up biopsies were performed in the opposite arm (biceps) at week 12 for the 50 mg/kg group and two placebo-treated patients and at week 24 for the 30 mg/kg group and two placebo-treated patients.

Patients continued weekly dosing with 30 or 50 mg/kg eteplirsen under Study 202 (Study no. 4658-us-202; ClinicalTrials.gov Identifier: NCT01540409) a long-term, open-label extension study. All efficacy assessments continued to be performed during Study 202, including a third biopsy (in the left deltoid muscle) in all patients at week 48. A voluntary fourth biopsy (11/12 patients) was performed at week 180. Monitoring of adverse events continued throughout the study. A schematic of the study design is shown in Fig. 1.

### Matched External Control Cohort

To compare the results of the eteplirsen cohort to a matched external control cohort, the Italian Telethon (n=97) and Leuven Neuromuscular Research Center (NMRC) (n=89) registries were used to compile control data. These registries were chosen because the following data was available: longitudinal 6MWT data in exon 51 amenable patients, longitudinal 6MWT data up to 36 months, and genetic mutation data. There was also an equivalent standard of care, including steroids, in these registries. Patient data was selected based on the following factors: baseline 6MTW and steroid treatment, age of 7 years or older, and amenable to exon 51 skipping (Fig. 2)

### Study Drug

Eteplirsen was supplied by Sarepta Therapeutics, Inc. in single-use vials of phosphate-buffered saline (100 mg/ml). Eteplirsen was reconstituted with 150 ml normal saline and infused over 60 minutes. Placebo, administered during the first 24 weeks of Study 201, was supplied as identical vials of phosphate-buffered saline and was administered in the same manner as eteplirsen.

### Safety and Tolerability Monitoring

Safety was assessed by evaluation of adverse events, vital signs, physical examinations, electrocardiograms, echocardiograms, and clinical laboratory testing. In addition, kidney function was monitored via regular assessments of serum cystatin C and urine cystatin C and KIM-1.

### Biochemical Efficacy Assessments

Dystrophin expression levels were determined in both the eteplirsen treated cohort and matched external control cohort, based on MANDYS106 [a gift from Glen Morris, MDA Monoclonal Antibody Library], a highly sensitive marker for dystrophin used in prior studies of eteplirsen and other exon skipping candidates. Three 10 µm frozen sections, separated by at least 200 µm, were stained with MANDYS106, followed by a secondary antibody (Alexa Fluor 594 goat antimouse antibody). Four pathologists reviewed the tissue blinded and reported percent dystrophin-positive fibers, which were calculated by dividing the number of positive fibers by the total fibers counted. As normal muscle samples have 100% dystrophin-positive fibers, percent dystrophin-positive fibers is expressed as a percentage of normal.

### Clinical Efficacy Assessments

The 6MWT was administered using the protocol established for patients with DMD by McDonald, et al. (Muscle Nerve, 2010; 42:966-74;Muscle Nerve, 2010; 41:500-10 herein incorporated by reference), except there was no use of the StepWatch actimetry device. Exploratory functional outcomes included the North Star Ambulatory Assessment, quantitative muscle testing, the 9-Hole Peg Test, pulmonary function testing (PFT), timed function tests, and assessment of quality of life.

### Example 1: Subject Characteristics

Baseline characteristics of the 12 patients in the eteplirsen treated cohort and of the 13 patients in the external matched control cohort are summarized in Table 1. Five different genotypes amenable to exon 51 skipping were represented in both the eteplirsen and control populations. Mean distances on the 6-Minute Walk Test (6MWT) at baseline were similar to those in other studies of children with DMD, and as expected, were well below the 600 plus meters typically observed in age-matched healthy children.

**Table 1. Baseline Demography and Disease Characteristics**

| | **Eteplirsen Study** | **External Control Groups** | |
|---|---|---|---|
| **PARAMETER** | **201/202 Study (n=12)** | **Exon 51 (n=13)** | **Any Exon (n=50)** |
| *Sex* | Male | Male | Male |
| *Mean age, yrs (SD)* | 9.4 (1.18) | 9.5 (1.45) | 9.7 (1.52) |
| *Mean 6MWT distance, m (SD* | 363.2 (42.19 | 357.6 (66.75) | 355.7 (87.28) |
| *Deletion mutations represented* | 45-50, 48-50, 49-50, 50, 52 | 45-50, 48-50, 49-50, 50, 52 | Skippable mutations |
| *Steroid use* | 100% | 100% | 100% |

| | | | |
|---|---|---|---|
| Abbreviations: 6MWT=6-Minute Walk Test; SD=standard deviation. | | | |

### Example 2: Safety and Lack of Adverse Events

Eteplirsen was well tolerated with no treatment-related adverse events, serious adverse events, discontinuations or missed doses through 216 weeks of treatment. Moreover, no clinically significant changes were observed on physical examination or in vital signs. Electrocardiograms, echocardiograms, and PFTs remained stable, and chemistries showed no clinically significant changes in hematologic, renal, coagulation or liver functions. Mild and transient proteinuria was observed in a single placebo-treated subject.

### Example 3: Natural History of External Control Cohort

Once the external control cohort was developed and patient data was characterized by age and being amenable to exon skipping, comparisons within the control cohort were made. Fig. 2 shows the derivation of matched external control groups by prospectively defined filters. Patients <7 years old and amenable to exon skipping (n=17) initially improved in walking ability through 24 months and then maintained 6MWT distance above baseline through 36 months. However, patients 7 years of age or older and amenable to exon skipping (n=50), had a significant decrease in 6MWT distance over 36 months. There was a statistically significant 194 m difference (p<0.001) between patients younger than 7 years old and patients 7 years or older (Fig. 3).

This is further evident when looking at trends in the patients of the external control cohort that have any genotype. Patients younger than 7 years old (n=25) initially improved in walking ability through 24 months and maintained 6MWT distance above baseline through 36 months, whereas patients 7 years of age or older (n=91) had a significant decrease in 6MWT distance through 36 months. The difference between the age groups was a statistically significant 167 m (p<0.001) (Fig. 4). These data indicate that ambulation declines much more rapidly in patients of seven years of age or older, relative to younger patients.

Beyond comparing the differences between age groups, a comparison was also made between mutations causing DMD in patients 7 years of age or older. As shown in Fig. 5, patients having a mutation amenable to exon 51 skipping had a greater loss in 6MWT distance over 36 months, as compared to patients having a mutation that is amenable to exon skipping, generally, or DMD patients having any mutation. This data shows that patients with exon 51 mutations decline more rapidly than any other genotypes.

### Example 4: Functional Outcomes

The progressive loss of walking ability is a universal hallmark of DMD, with most patients showing functional compromise by 7 or 8 years of age and becoming wheelchair dependent by 10 to 14 years of age. Consistent with this, data from the matched external control patients show a decline in walking ability over 36 months, culminating in a loss of approximately 250 meters. In marked contrast, eteplirsen-treated patients maintained a stable walking distance over the duration of the study (Fig. 6). The difference between the eteplirsen-treated patients and those in the matched external control cohort was also evident when analyzing the 6MWT distance by the percent change from baseline. A -74% decline was observed in the external control patients (at month 36), whereas there was only a -42% decline in the eteplirsen-treated patients at 42 months. When the placebo-delayed eteplirsen-treated patients were excluded (n=8), a -43% decline was observed at 48 months of treatment (Fig. 7). As shown in Fig. 7, unexpectedly eteplirsen treatment stabilized patient ambulation between weeks 168 to 192 (42 months to 48 months), which, as described below, was also associated with continued stable pulmonary function and dystrophin expression.

The benefit of eteplirsen treatment was further evident by looking at the cumulative ambulation loss over 36 months. Six of the thirteen patients in the matched external control cohort lost ambulation, whereas only two of the twelve patients in the eteplirsen cohort loss ambulation (Fig. 8). Overall, 46.2% of the matched external control cohort loss ambulation and only 16.7% in the eteplirsen cohort. And, as noted above, 10/12 eteplirsen-treated patients were ambulant at 192 weeks of treatment.

### Example 5: Efficacy Compared to Matched External Control Cohort

After 180 weeks, eteplirsen-treated patients had a significantly higher amount of dystrophin positive fibers compared to the matched external control patients as determine by immunohistochemistry. Four independent pathologists that were blinded each found a statistically significant higher percentage of dystrophin positive fibers in the eteplirsen-treated patients (p<0.001) (Fig. 9).

### Example 6: Pulmonary Function

Respiratory muscle function from baseline through Week 192 was determined in both cohorts, as measured by maximum inspiratory and expiratory pressure (MIP and MEP), and forced vital capacity (FVC) (Fig. 10). The eteplirsen-treated patients either maintained or had only a slight decline in all of MIP, MEP, and FVC over the duration of the study. The matched external control patients, however, had a steady decline in all three measurements over time. This indicates that eteplirsen is able to prevent the loss of pulmonary function over time.

### Example 7: Extended Measurement of Functional Outcomes

The ambulatory ability of eteplirsen-treated patients was further evaluated at 216 weeks and compared to the external control subjects, as carried out in Example 4. Table 2 shows the results of the 6MWT displayed as 6MWD in meters, at 216 weeks in the eteplirsen-treated cohort. Table 3 shows the results of the 6MWT at 216 week in the matched external control cohort.

**Table 2: Study 201/202 Subjects (n=12) 6MWT Results at Year 4**

| **Dose Group** | **Subject Number** | **Baseline 6MWT/6MWD** | **Year 3 6MWT/6MWD** | **Year 4 6MWT/6MWD** | **Age at year 4** |
|---|---|---|---|---|---|
| Placebo to 30 mg/kg | 007 | 374 | 312 | 197 | 11.7 |
| | 008 | 346 | 100 | 55 | 14.2 |
| Placebo to 50 mg/kg | 005 | 374 | 247 | 143 | 11.7 |
| | 013 | 400 | 301 | 230 | 14.3 |
| 30 mg/kg | 002 | 416 | 378 | 349 | 12.7 |
| | 006 | 355 | 359 | 332 | 14.2 |
| | 009 | 330 | 0 | 0 | 13.9 |
| | 010 | 256 | 0 | 0 | 13.9 |
| 50 mg/kg | 003 | 366 | 324 | 192 | 11.0 |
| | 004 | 389 | 355 | 221 | 12.5 |
| | 012 | 351 | 298 | 237 | 14.6 |
| | 015 | 401 | 483 | 400 | 13.3 |

**Table 3: Matched External Control Cohort (n=13) 6MWT Results at Year 4**

| **Subject Number** | **Baseline 6MWT/6MWD** | **Year 3 6MWT/6MWD** | **Year 4 6MWT/6MWD** | **Age at Year 4** |
|---|---|---|---|---|
| MI15** | 380 | 195 | *Missing | 12.6 |
| OBG16 | 295 | 35 | 0 | 13.3 |
| OBG20 | 380 | 0 | 0 | 12.0 |
| NA7 | 329 | 218 | 0 | 12.8 |
| TO6 | 388 | 0 | 0 | 12.1 |
| TO2 | 388 | 0 | 0 | 14.1 |
| PI3 | 325 | 0 | 0 | 11.3 |
| PV12 | 458 | 362 | 300 | 14.2 |
| Ge10 | 200 | 0 | 0 | 15.5 |
| MIBESTA8 | 373 | 273 | 0 | 13.0 |
| BS | 327 | 0 | 0 | 15.5 |
| KB | 451 | 240 | 0 | 15.2 |
| SL** | 355 | *Missing | *Missing | 12.3 |

| | | | | |
|---|---|---|---|---|
| *External control patients with missing 6MWT data at year 4 ** Of note, patients MI15 and SL were subsequently reported to have loss of ambulation with "0" meters on the 6MWT at ∼4.5 years. In addition, external control patient PV12 was known to have lost ambulation with a 6MWT of "0" at 4.8 years. | | | | |

The results of the 6MWT in the eteplirsen-treated cohort and the external matched control cohort were compared and analyzed for statistically significant differences. Consistent with the data described in Example 4, there was a significant difference in mean change from baseline between the eteplirsen-treated cohort and the external matched control cohort as determined by MMRM (mixed model for repeated measurement) and ANCOVA (analysis of covariance), summarized in Table 4. For the MMRM method, data from all 12 patients in the eteplirsen-treated cohort and all 13 patients from the external matched control cohort was used. For the ANCOVA method, data from all 12 patients in the eteplirsen-treated cohort and 11 patients from the external matched cohort (excluded patients with missing data) was used.

**Table 4: Summary of Year 4 6MWT Difference in Mean Change**

| **Model** | **MMRM** | **ANCOVA** |
|---|---|---|
| Covariate: Baseline 6MWT | 146.5, *p*=0.003 | 150.3, *p*=0.002 |
| Covariates: Baseline 6MWT, Age | 143.1, *p*=0.004 | 156.1, *p*=0.002 |
| Rank transformed data analyzed Covariate: | 8.4, *p*=0.004 | 8.7, *p*=0.001 |

Moreover, as set forth in Fig. 12, the difference between the mean change from baseline between the eteplirsen-treated patient cohort as compared to the external matched control cohort increased from 151 meters at 3 years to 162 meters at year 4. These data provide further evidence that eteplirsen treatment slows disease progression over time in DMD patients.

The benefit of eteplirsen treatment was further evident by looking at the cumulative ambulation loss over 216 weeks. Ten of the thirteen patients in the matched external control cohort lost ambulation, whereas only two of the twelve patients in the eteplirsen cohort lost ambulation (Fig. 11). All ten of the eteplirsen-treated patients that were ambulant at 192 weeks remained able to walk at 216 weeks. Overall, 89.7% of the matched external control cohort loss ambulation and only 16.7% in the eteplirsen cohort. See Fig. 13, which sets forth the percentage of non-ambulant patients in each cohort by time. The median age for loss of ambulation was 12.8 years in the external matched control cohort, whereas the age for loss of ambulation in the eteplirsen-treated cohort could not be estimated. This data provided further support that the eteplirsen-treated patients performed better than the external control matched patients in a statistically significant manner.

### Example 8: Efficacy in Patients Who Have Lost Ability to Rise Independently from Supine

Some literature suggests an association in DMD patients between loss of ability to rise from the floor independently and a subsequent loss of ambulation within the following year. As shown in Table 5 below, four eteplirsen-treated patients lost the ability to rise without external support from years 1 to 3. Yet, despite the loss of ability to rise from supine, these patients treated on eteplirsen remain ambulant at year 4.

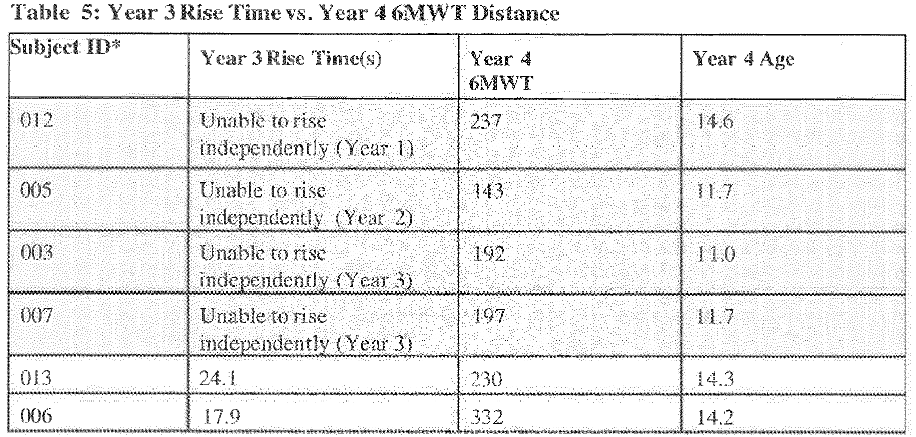

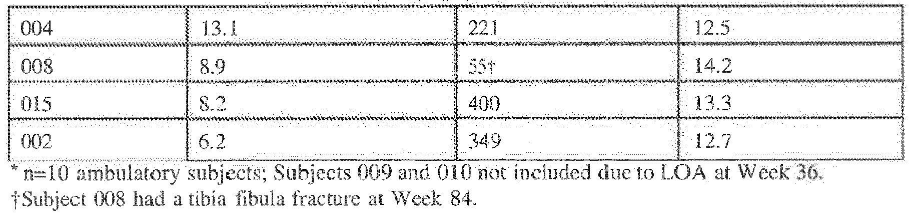

These data indicate that eteplirsen-treatment preserves ambulatory ability, as measured by the 6MWT, even in patients who have lost the ability to rise independently from supine.

### Example 9. Eteplirsen and Intensity of Steroid Use

Steroid use has been shown to impact disease progression in DMD patients. Importantly, the eteplirsen-treated patients, as well as all external controls, were on a stable dose of steroids at least six months prior to enrollment, and remained on steroids throughout the study.

Deflazacort and prednisone were used in equal proportion by eteplirsen and external control patients. Fig. 14 depicts the number of patients receiving deflazacort or prednisone by continuous or intermittent schedule. Within each steroid regimen, a calculation of the percent of mean recommended steroid dose is shown. The external control group actually received a higher percentage of the standard recommended dose established by the CDC/TREAT-NMD guidelines (ranging from 88 to 100%) compared to the eteplirsen-treated patients (ranging from 75 to 79%).

### Example 10. Ambulation Preserved in Eteplirsen Patients with Decreases in North Star Ambulatory Assessment (NSAA) Scores

Similar to the 6MWT results described above, eteplirsen-treated patients demonstrated a slower rate of decline in NSAA score at 2 and 3 years following treatment, resulting in a difference of 2.6 points at 3 years compared to the external control group cohort. This result represents the preservation of one or more activities of daily living. Two patients at year 3 in the eteplirsen treated group (003 and 005) fell below the NSAA threshold expected to predict loss of ambulation in one year [Ricotti et al. (2015) J Neurol Neurosurg Psychiatry 0:1-7], yet both maintained ambulation at year 4 (of 143 and 192 meters, respectively). See Table 6 below.

**Table 6. Year 3 NSAA vs. Year 4 6MWT**

| **Subject ID*** | **Year 3 NSAA Score** | **Year 4 6MWT Distance (m)** | **Year 4 Age** |
|---|---|---|---|
| 003 | 7 | 192 | 11.0 |
| 005 | 8 | 143 | 11.7 |
| 012 | 11 | 237 | 14.6 |
| 007 | 13 | 197 | 1 1.7 |
| 008 | 13 | 55† | 14.2 |
| 006 | 13 | 332 | 14.2 |
| 013 | 14 | 230 | 14.3 |
| 004 | 24 | 221 | 12.5 |
| 015 | 25 | 400 | 13.3 |
| 302 | 25 | 349 | 12.7 |

| | | | |
|---|---|---|---|
| *n= 10 ambulatory subjects; Subjects 009 and 010 not included due to LOA at Week 36. † Subject 008 had a tibia fibula fracture at Week 84. | | | |

All publications and patent applications cited in this specification are herein incorporated by reference as if each individual publication or patent application were specifically and individually indicated to be incorporated by reference.

### REFERENCES

1. Emery AEH. Population frequencies of inherited neuromuscular diseases - a world survey. Neuromuscul Disord 1991;1:19-29.
2. Mendell JR, Shilling C, Leslie ND, Flanigan KM, al-Dahhak R, Gastier-Foster, J, et al. Evidence-based path to newborn screening for Duchenne muscular dystrophy. Ann Neurol 2012;71:304-13.
3. McDonald CM, Abresch RT, Carter GT, Fowler WM Jr, Johnson ER, Kilmer DD, et al. Profiles of neuromuscular diseases. Duchenne muscular dystrophy. Am J Phys Med Rehabil 1995;74:S70-S92.
4. Bushby K, Finkel R, Birnkrant DJ, Case LE, Clemens PR, Cripe L, et al. Diagnosis and management of Duchenne muscular dystrophy, part 1: diagnosis, and pharmacological and psychosocial management. Lancet Neurol 2010;9:77-93.
5. Kohler M, Clarenbach CF, Böni L, Brack T, Russi EW, Bloch KE. Quality of life, physical disability and respiratory impairment in Duchenne muscular dystrophy. Am J Respir Crit Care Med 2005;172:1032-6.
6. Mendell JR, Moxley RT, Griggs RC, Brooke MH, Fenichel GM, Miller JP, et al. Randomized, double-blind six-month trial of prednisone in Duchenne's muscular dystrophy. N Engl J Med 1989;320:1592-97.
7. Manzur AY, Kuntzer T, Pike M, Swan A. Glucocorticoid corticosteroids for Duchenne muscular dystrophy. Cochrane Database Syst Rev. 2004;(2):CD003725.
8. van Deutekom JC, Janson AA, Ginjaar IB, Frankhuizen WS, Aartsma-Rus A, Bremmer-Bout M, et al. Local dystrophin restoration with antisense oligonucleotide PR0051. N Engl J Med 2007;357:2677-86.
9. Kinali M, Arechavala-Gomeza V, Feng L, Cirak S, Hunt D, Adkin C, et al. Local restoration of dystrophin expression with the morpholino oligomer AVI-4658 in Duchenne muscular dystrophy: a single-blind, placebo-controlled, dose-escalation, proof-of-concept study. Lancet Neurol 2009;8:918-28.
10. Goemans NM, Tulinius M, van den Akker JT, Burm BE, Ekhart PF, Heuvelmans N, et al. Systemic administration of PR0051 in Duchenne's muscular dystrophy. N Engl J Med 2011;364:1513-22.
11. Cirak S, Arechavala-Gomeza V, Guglieri M, Feng L, Torelli S, Anthony K, et al. Exon skipping and dystrophin restoration in patients with Duchenne muscular dystrophy after systemic phosphorodiamidate morpholino oligomer treatment: an open-label, phase 2, dose-escalation study. Lancet 2011;378:595-605.
12. Aartsma-Rus A, Fokkema I, Verschuuren J, Ginjaar I, van Deutekom J, van Ommen GJ, et al. Theoretic applicability of antisense-mediated exon skipping for Duchenne muscular dystrophy mutations. Hum Mutat 2009;30:293-99.
13. Muntoni F, Torelli S, Ferlini A. Dystrophin and mutations: one gene, several proteins, multiple phenotypes. Lancet Neurol. 2003;2:731-40.
14. Bushby KM, Gardner-Medwin D. The clinical, genetic and dystrophin characteristics of Becker muscular dystrophy. I. Natural history. J Neurol 1993;240:98-104.
15. Arechavala-Gomeza V, Graham IR, Popplewell LJ, Adams AM, Aartsma-Rus A, Kinali M, et al. Comparative analysis of antisense oligonucleotide sequences for targeted skipping of exon 51 during dystrophin pre-mRNA splicing in human muscle. Hum Gene Ther 2007;18:798-810.
16. Mendell JR, Campbell K, Rodino-Klapac L, Sahenk Z, Shilling C, Lewis S, et al. Dystrophin immunity revealed by gene therapy in Duchenne muscular dystrophy. N Engl J Med 2010;363:1429-37.
17. Nguyen TM, Morris GE. Use of epitope libraries to identify exon-specific monoclonal antibodies for characterization of altered dystrophins in muscular dystrophy. Am J Hum Genet 1993;52:1057-66.
18. Arechavala-Gomeza V, Kinali M, Feng L, Brown SC, Sewry C, Morgan JE, et al. Immunohistological intensity measurements as a tool to assess sarcolemma-associated protein expression. Neuropathol Appl Neurobiol 2010;36: 265-74.
19. McDonald CM, Henricson EK, Han JJ, Abresch RT, Nicorici A, Elfring GL, et al. The 6-minute walk test as a new outcome measure in Duchenne muscular dystrophy. Muscle Nerve 2010;41:500-10.
20. Mazzone E, Vasco G, Sormani MP, Torrente Y, Berardinelli A, Messina S, et al. Functional changes in Duchenne muscular dystrophy: a 12-month longitudinal cohort study. Neurology 2011;77(3):250-6.
21. McDonald CM, Henricson EK, Han JJ, Abresch RT, Nicorici A, Atkinson L, et al. The 6-minute walk test in Duchenne/Becker muscular dystrophy: longitudinal observations. Muscle Nerve 2010;42: 966-74.
22. Strober JB. Therapeutics in Duchenne muscular dystrophy. NeuroRX 2006;3:225-34.
23. Hoffman EP, Fischbeck KH, Brown RH, Johnson M, Medori R, Loike JD, et al. Characterization of dystrophin in muscle-biopsy specimens from patients with Duchenne's or Becker's muscular dystrophy. N Engl J Med 1988;318:1363-68.
24. Azofeifa J, Voit T, Hubner C, Cremer M. X-chromosome methylation in manifesting and healthy carriers of dystrophinopathies: concordance of activation ratios among first degree female relatives and skewed inactivation as cause of the affected phenotypes. Hum Genet 1995;96:167-76.
25. van Putten M, Hulsker M, Nadarajah VD, van Heiningen SH, van Huizen E, van Iterson M, et al. The Effects of Low Levels of Dystrophin on Mouse Muscle Function and Pathology. PLoS ONE 2012;7:e31937.
26. Brooke MH, Fenichel GM, Griggs RC, Mendell JR, Moxley R, Miller JP, et al. Clinical investigation in Duchennc dystrophy: 2. Determination of the "power" of therapeutic trials based on the natural history. Muscle Nerve. 1983;6:91-103.
27. Ahmad A, Brinson M, Hodges BL, Chamberlain JS, Amalfitano A. Mdx mice inducibly expressing dystrophin provide insights into the potential of gene therapy for Duchenne muscular dystrophy. Hum Mol Genet 2000;9:2507-15.
28. Hoffman EP, Bronson A, Levin AA, Takeda S, Yokota T, Baudy AR, Connor EM.. Restoring dystrophin expression in Duchenne muscular dystrophy muscle: Progress in exon skipping and stop codon read through. Am J Path 2011; 179:12-22.
29. Merlini L, Gennari M, Malaspina E, Cecconi I, Armaroli A, Gnudi S, et al. Early corticosteroid treatment in 4 Duchenne muscular dystrophy patients: 14-year follow-up. Muscle Nerve 2012;45:796-802.
30. Fletcher S, Honeyman K, Fall AM, Harding PL, Johnsen RD, Steinhaus JP, et al. Morpholino oligomer-mediated exon skipping averts the onset of dystrophic pathology in the mdx mouse. Mol Ther 2007;15:1587-92.
31. Yokota T, Lu QL, Partridge T, Kobayashi M, Nakamura A, Takeda S,, et al. Efficacy of systemic morpholino exon-skipping in Duchenne dystrophy dogs. Ann Neurol 2009;65:667-76.
32. Aartsma-Rus A, Janson AA, Kaman WE, Bremmer-Bout M, van Ommen GJ, den Dunnen JT, et al. Antisense-induced multiexon skipping for Duchenne muscular dystrophy makes more sense. Am J Hum Genet 2004;74:83-92.

The present disclosure will now be further defined in the following paragraphs:
1. A method for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly for more than 168 weeks, such that disease progression in the patient is delayed as measured by the 6 Minute Walk Test (6MWT), thereby treating the patient.
2. A method for maintaining ambulation, or reducing the loss of ambulation, in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly for more than 168 weeks, thereby maintaining ambulation, or reducing the loss of ambulation relative to baseline, in the patient as measured by the 6 Minute Walk Test (6MWT).
3. A method for maintaining pulmonary function or reducing loss of pulmonary function in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly for more than 168 weeks, thereby maintaining pulmonary function; or reducing the loss of pulmonary function, in the patient relative to baseline.
4. A method for restoring an mRNA reading frame to induce dystrophin protein production in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly for more than 168 weeks, thereby restoring the mRNA reading frame to induce dystrophin protein production in the patient.
5. A method for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly, such that disease progression in the patient is delayed as measured by the 6 Minute Walk Test (6MWT), thereby treating the patient, wherein the patient has lost the ability to rise independently from supine prior to treatment with eteplirsen.
6. A method for treating a patient with Duchenne muscular dystrophy (DMD) in need thereof who has a mutation of the DMD gene that is amenable to exon 51 skipping by delaying disease progression, comprising intravenously administering to the patient eteplirsen at a dose of 30 mg/kg weekly, such that disease progression in the patient is delayed as measured by the 6 Minute Walk Test (6MWT), thereby treating the patient, wherein the patient loses the ability to rise independently from supine during treatment with eteplirsen.
7. A method for maintaining ambulation, or reducing the loss of ambulation, in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby maintaining ambulation, or reducing the loss of ambulation relative to baseline, in the patient as measured by the 6 Minute Walk Test (6MWT), wherein the patient has lost the ability to rise independently from supine prior to treatment with eteplirsen.
8. A method for maintaining ambulation, or reducing the loss of ambulation, in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby maintaining ambulation, or reducing the loss of ambulation relative to baseline, in the patient as measured by the 6 Minute Walk Test (6MWT), wherein the patient loses the ability to rise independently from supine during treatment with eteplirsen.
9. A method for maintaining pulmonary function or reducing loss of pulmonary function in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby maintaining pulmonary function, or reducing the loss of pulmonary function, in the patient relative to baseline, wherein the patient has lost the ability to rise independently from supine prior to treatment with eteplirsen.
10. A method for maintaining pulmonary function or reducing loss of pulmonary function in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby maintaining pulmonary function, or reducing the loss of pulmonary function, in the patient relative to baseline, wherein the patient loses the ability to rise independently from supine during treatment with eteplirsen.
11. A method for restoring an mRNA reading frame to induce dystrophin protein production in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby restoring the mRNA reading frame to induce dystrophin protein production in the patient, wherein the patient has lost the ability to rise independently from supine prior to treatment with eteplirsen
12. A method for restoring an mRNA reading frame to induce dystrophin protein production in a patient with Duchenne muscular dystrophy (DMD) in need thereof who has a mutation of the DMD gene that is amenable to exon 51 skipping, comprising intravenously administering to the patient eteplirsen at a dose of 30 mg/kg weekly, thereby restoring the mRNA reading frame to induce dystrophin protein production in the patient, wherein the patient loses the ability to rise independently from supine during treatment with eteplirsen.
13. Eteplirsen for use in treating Duchenne muscular dystrophy (DMD) in a patient in need thereof, the patient having a mutation of the DMD gene that may be corrected by skipping exon 51, wherein the treatment comprises administering to the patient a single intravenous dose of eteplirsen of 30 mg/kg once a week, thereby delaying the progression of the disease over 168 weeks as determined by the 6 Minute Walk Test (6MWT)
14. Eteplirsen for use in maintaining ambulation, or reducing loss of ambulation in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby maintaining ambulation or reducing loss of ambulation relative to baseline over 168 weeks as determined by the 6 Minute Walk Test (6MWT)
15. Eteplirsen for use in maintaining pulmonary function or reducing loss of pulmonary function in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby maintaining pulmonary function or reducing the loss of pulmonary function relative to baseline over 168 weeks.
16. Eteplirsen for use in restoring an mRNA reading frame to induce dystrophin protein production in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby restoring the mRNA reading frame to induce dystrophin protein production over 168 weeks.
17. Eteplirsen for use in restoring an mRNA reading frame to induce dystrophin protein production in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby restoring the mRNA reading frame to induce dystrophin protein production for at least 216 weeks.
18. The method of any one of paragraphs 1-12, wherein eteplirsen is intravenously administered to the patient weekly for at least 180 weeks.
19. The method of any one of paragraphs 1-12, wherein eteplirsen is intravenously administered to the patient weekly for at least 192 weeks
20. The method of any one of paragraphs 1-12, wherein eteplirsen is intravenously administered to the patient weekly for more than 192 weeks
21. The method of any one of paragraphs 1-12, wherein eteplirsen is intravenously administered to the patient weekly for at least 216 weeks.
22. The method of any one of paragraphs 3, 9, 10, or use of paragraph 15, wherein pulmonary function is measured by Maximum Expiratory Pressure (MEP), Maximum Inspiratory Pressure (MIP), or Forced Vital Capacity (FVC).
23. The method of any one of paragraphs 4, 11, 12, or use of paragraphs 16 or 17, wherein the dystrophin protein production is measured by reverse transcription polymerase chain reaction (RT-PCR), western blot analysis, or immunohistochemistry (IHC).
24. The method of any one of paragraph 20 or 21, wherein the patient maintains a 6 Minute Walk Distance of at least 55 meters, at least 100 meters, at least 200 meters, at least 300 meters, or at least 400 meters, at 216 weeks of treatment.
25. The method of any one of paragraphs 1-12 or 18-21, wherein eteplirsen is administered as an intravenous infusion over 35 to 60 minutes.
26. The method of any one of paragraphs 1-12 or 18-21, further comprising confirming that the patient has a mutation in the DMD gene that is amenable to exon 51 skipping prior to administering eteplirsen.

## Claims

1. Eteplirsen for use in treating Duchenne muscular dystrophy (DMD) in a patient in need thereof, the patient having a mutation of the DMD gene that may be corrected by skipping exon 51, wherein the treatment comprises administering to the patient a single intravenous dose of eteplirsen of 30 mg/kg once a week, thereby delaying the progression of the disease over 168 weeks as determined by the 6 Minute Walk Test (6MWT).

2. Eteplirsen for use in maintaining ambulation, or reducing loss of ambulation in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby maintaining ambulation or reducing loss of ambulation relative to baseline over 168 weeks as determined by the 6 Minute Walk Test (6MWT).

3. Eteplirsen for use in maintaining pulmonary function or reducing loss of pulmonary function in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby maintaining pulmonary function or reducing the loss of pulmonary function relative to baseline over 168 weeks.

4. Eteplirsen for use in restoring an mRNA reading frame to induce dystrophin protein production in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby restoring the mRNA reading frame to induce dystrophin protein production over 168 weeks.

5. Eteplirsen for use in restoring an mRNA reading frame to induce dystrophin protein production in a patient having Duchenne muscular dystrophy (DMD), the patient having a mutation of the DMD gene that may be corrected by skipping exon 51, wherein the treatment comprises administering to the patient a single intravenous dose of eteplirsen of 30 mg/kg once a week, thereby restoring the mRNA reading frame to induce dystrophin protein production for at least 216 weeks.

6. Eteplirsen for use of any one of claims 1-5, wherein eteplirsen is intravenously administered to the patient weekly for at least 180 weeks.

7. Eteplirsen for use of any one of claims 1-5, wherein eteplirsen is intravenously administered to the patient weekly for at least 192 weeks.

8. Eteplirsen for use of any one of claims 1-5, wherein eteplirsen is intravenously administered to the patient weekly for more than 192 weeks.

9. Eteplirsen for use of any one of claims 1-5, wherein eteplirsen is intravenously administered to the patient weekly for at least 216 weeks.

10. Eteplirsen for use of claim 3, wherein pulmonary function is measured by Maximum Expiratory Pressure (MEP), Maximum Inspiratory Pressure (MIP), or Forced Vital Capacity (FVC).

11. Eteplirsen for use of claim 4 or 5, wherein the dystrophin protein production is measured by reverse transcription polymerase chain reaction (RT-PCR), western blot analysis, or immunohistochemistry (IHC).

12. Eteplirsen for use of claim 1 or 2, wherein the patient maintains a 6 Minute Walk Distance of at least 55 meters, at least 100 meters, at least 200 meters, at least 300 meters, or at least 400 meters, at 216 weeks of treatment.

13. Eteplirsen for use of any one of claims 1-12, wherein eteplirsen is administered as an intravenous infusion over 35 to 60 minutes.

14. Eteplirsen for use of any one of claims 1-13, further comprising confirming that the patient has a mutation in the DMD gene that is amenable to exon 51 skipping prior to administering eteplirsen.
